(19) Europäisches Patentamt
European Patent Office
Office européen des brevets

(11) EP 1 946 779 A1

(12) **EUROPEAN PATENT APPLICATION**

(43) Date of publication:
**23.07.2008 Bulletin 2008/30**

(51) Int Cl.:
***A61K 45/06*** *(2006.01)* ***A61P 3/06*** *(2006.01)*

(21) Application number: **07384009.2**

(22) Date of filing: **16.01.2007**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Laboratorios del Dr. Esteve S.A.**
**08041 Barcelona (ES)**

(72) Inventors:
- **Fisas-Escasany, Maria Angeles**
  **08025 Barcelona (ES)**
- **Buschmann, Helmut H.**
  **08960 Sant Just Desvem (ES)**

(74) Representative: **Peters, Hajo**
**Bosch Graf von Stosch Jehle Patentanwaltsgesellschaft mbH**
**Flüggenstrasse 13**
**80639 München (DE)**

(54) **Combination of substituted pyrazolines and agent for treating dyslipidemia**

(57) The present invention relates to an active substance combination comprising at least one substituted pyrazoline compound and at least one lipid lowering compound used to treat dyslipidaemia, a medicament comprising said active substance combination, a pharmaceutical formulation comprising said active substance combination and the use of said active substance combination for the manufacture of a medicament.

EP 1 946 779 A1

## Description

The present invention relates to an active substance combination comprising at least one substituted pyrazoline compound and at least one lipid lowering compound, a medicament comprising said active substance combination, a pharmaceutical formulation comprising said active substance combination and the use of said active substance combination for the manufacture of a medicament, more particularly for the manufacture of a medicament useful to treat dyslipidaemia.

The conversion of 3-hydroxy-3-methylglutaryl-coenzyme A (HMG-CoA) to mevalonate is an early step in the biosynthetic pathway of cholesterol. This step is catalyzed by the enzyme HMG-CoA reductase. Statins inhibit HMG-CoA reductase from catalyzing this conversion. Statins, as such, are quite potent lipid lowering agents and are useful for the prophylaxis and/or treatment of cardiovascular diseases. A safety concern related to the use of statins is the development of rhabdomyolysis, the pathological breakdown of skeletal muscle, which may lead to acute renal failure when muscle breakdown products damage the kidney. Consequently, there is still a big demand for potent therapeutic agents to treat dyslipidaemia, possibly, showing less incidents of undesired side effects of statins, or at least less pronounced.

The term "dyslipidaemia" is defined both as a disease entity in its own right and as a component of the Metabolic Syndrome. Thus, within the context of the various definitions of the Metabolic Syndrome, certain lipid abnormalities are specifically defined as follows :

<u>According to world Health Organisation (WHO) (1999) :</u>

- Raised plasma triglycerides (≥1.7 mmol/L; 150 mg/dL).
- Low plasma HDL-cholesterol (< 0.9 mmol/L, 35 m/dL men; < 1.0 mmol/L, 39 mg/dL women).

<u>According to National Cholesterol Education Programme (NCEP) Adult Treatment Panel (ATP) III (2002) :</u>

- Raised plasma triglycerides (≥1.7 mmol/L; 150 mg/dL).
- Low plasma HDL-cholesterol (< 1.03 mmol/L, 40 mg/dL men; < 1.29 mmol/L, 50 mg/dL women).

<u>According to International Diabetes Federation (IDF) (2005):</u>

- Raised serum triglycerides (≥1.7 mmol/L; 150 mg/dL) or specific treatment for this lipid abnormality.
- Reduced plasma HDL-cholesterol (< 1.03 mmol/L, 40 mg/dL men; 1.29 mmol/L, 50 mg/dL women) or specific treatment for this abnormality.

In addition to the above lipid abnormalities pertaining to definitions of the Metabolic Syndrome, "dyslipidaemia" also encompasses the following disorders as described by the NCEP in their ATP III Guidelines (NCEP ATP III, 2002): heterozygous familial hypercholesterolaemia (FH), homozygous familial hypercholesterolaemia (FH), familial defective apolipoprotein B-100 (FDB), polygenic hypercholesterolaemia hypertriglyceridaemia including familial combined hyperlipidaemia, familial hypertriglyceridaemia and familial dysbetalipoproteinaemia, low HDL-cholesterol (with or without hypertriglyceridaemia), diabetic dyslipidaemia (ie atherogenic dyslipidaemia in persons with Type 2 diabetes), elevated LDL-cholesterol and atherogenic dyslipidaemia. Other secondary dyslipidaemias include, but are not limited to, those evoked by hypothyroidism, nephrotic syndrome and other renal disorders, obstructive liver disease and protease-inhibitor induced dyslipidaemia.

As also stated in the NCEP ATP III Guidelines (2002), the necessity to treat dyslipidaemia in patients is defined not only by the serum or plasma concentrations of individual lipids, but also by the coexistence of lipid abnormalities with other cardiovascular risk factors and/or related disorders, eg hypertension, cardiovascular disease and Type 2 diabetes, the patient's medical history, also whether or not the patient has a history of cerebrovascular or cardiovascular adverse events, eg myocardial infarction, congestive heart failure, angina and/or haemorrhagic or thromboembolic stroke. Such clinical factors are well known to those skilled in the art and are taken into account in the decision whether or not to prescribe medications to treat dyslipidaemia.

In the present invention, all of the above indications are included under the term "dyslipidaemia".

It is also well known that obesity and visceral adiposity are important metabolic consequences of the excessive consumption of highly palatable, calorie-dense foods (Zephier et al, 1997; International Diabetes Federation, 2005; Grundy, 2004; Deedwania, 2004), and in some instances, from cravings for and binge eating, of specific palatable food sources (White et al, 2002; Phelan & Whadden, 2002; Yanovski, 2003; White & Grilo, 2005).

Dyslipaemia, with or without obesity, is major cause of insulin resistance and a key driver of the progression of pre-diabetes (insulin resistance and/or impaired glucose tolerance) to Type 2 diabetes (Boden & Laakso, 2004; Bays et al, 2004; IDF, 2005). Furthermore, there is also a high degree of association between Type 2 diabetes and dyslipidaemia

whereby the latter is characterised by raised plasma levels of small, dense LDL-cholesterol particles, elevated triglycerides and low concentrations of HDL-cholesterol particles (Boden & Laakso, 2004).

The Metabolic Syndrome consists of a cluster of cardio-metabolic risk factors, but obesity, central adiposity, lipid abnormalities, (raised serum triglycerides, low serum HDL-cholesterol) and impaired glucose tolerance or Type 2 diabetes are core symptoms of the Metabolic Syndrome, irrespective of whether a diagnosis of is made according to the criteria defined by the World Health Organisation (WHO, 1999), the National Cholesterol Education Programme - Third Adult Treatment Panel (NCEP ATP III, 2001) or the International Diabetes Federation (IDF, 2005). The cafeteria dietfed obese rats employed in these experiments are obese, dyslipidaemic and insulin resistant making them an excellent surrogate model for the human Metabolic Syndrome resulting from the over-consumption of highly palatable food sources.

It has now surprisingly been found that a lipid lowering compounds are able to be used in combination with one or more substituted pyrazoline compounds of general formula I and/or I' given below, in order to provide a medicament suitable for the prophylaxis and/or treatment of dyslipidaemia. This association reduces undesired side effects observed for example with statins. It has the further advantage to allow lower dosage of said lipid lowering compounds as compared to their doses when used alone. Finally the presently claimed combination exhibits a synergistic advantage in the treatment of metabolic disorders including, but not limited to, dyslipidaemia and/or obesity and/or appetency disorders and/or pre-diabetes (insulin resistance, impaired glucose tolerance) or Type 2 diabetes and/or Metabolic Syndrome, including drug-induced weight-gain or drug-induced obesity.

Consequently the present invention relates to an active substance combination comprising at least 1 compound selected from substituted pyrazolines of the general formula given below together with at least one "lipid lowering compound" used to treat dyslipidaemia, a medicament comprising said active substance combination, a pharmaceutical formulation comprising said active substance combination, and the use of said active substance combination for the manufacture of a medicament. This application also encompass the use of said active substance combination, a pharmaceutical formulation comprising said active substance combination and the use of said active substance combination for the manufacture of a medicament for the treatment of metabolic disorders including, but not limited to, dyslipidaemia and/or obesity and/or appetency disorders and/or pre-diabetes (insulin resistance, impaired glucose tolerance) or Type 2 diabetes and/or Metabolic Syndrome, including drug-induced weight-gain or drug-induced obesity.

In one of its aspects the present invention relates to an active substance combination comprising

(A) at least one substituted pyrazoline compound of general formula I

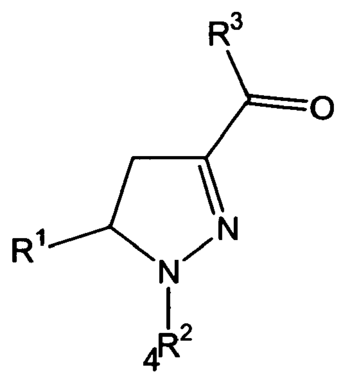

wherein

$R^1$ represents an optionally at least mono-substituted phenyl group;

$R^2$ represents an optionally at least mono-substituted phenyl group;

$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an $-NR^4R^5$-moiety,

$R^4$ and $R^5$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted

aryl or heteroaryl group, which may be condensed with an optionally at least mono-substituted mono- or polycyclic ring system and/or bonded via a linear or branched alkylene group; an $-SO_2-R^6$-moiety; or an $-NR^7R^8$-moiety, with the proviso that $R^4$ and $R^5$ do not identically represent hydrogen;

$R^6$ represents a linear or branched, saturated or unsaturated, optionally at least mono-substituted aliphatic group; a saturated or unsaturated, optionally at least mono-substituted, optionally at least one heteroatom as ring member containing cycloaliphatic group, which may be condensed with a mono- or polycyclic ring-system; or an optionally at least mono-substituted aryl or heteroaryl group, which may be condensed with a mono- or polycyclic ring system and/or bonded via a linear or branched alkylene group;

$R^7$ and $R^8$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,

and/or

at least one substituted pyrazoline compound of general formula I',

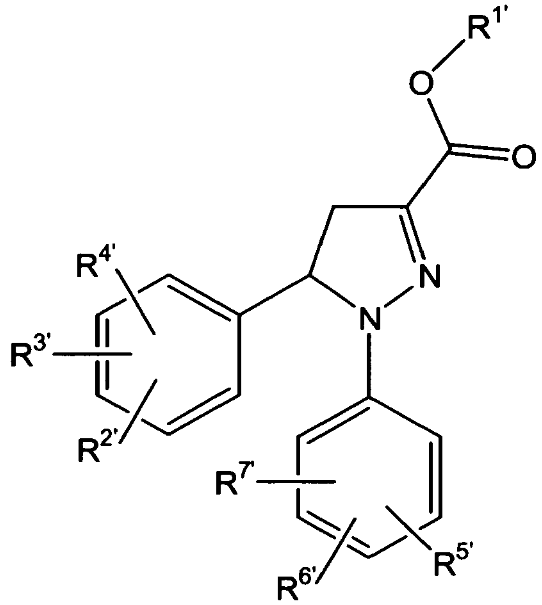

**I'**

wherein

$R^{1'}$ represents hydrogen or a linear or branched $C_{1-4}$-alkyl group,

$R^{2'}$, $R^{3'}$ and $R^{4'}$ independently of each other represent hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{8'}$, SH, $SR^{8'}$, $SOR^{8'}$, $SO_2R^{8'}$, $NH_2$, $NHR^{8'}$, $NR^{8'}R^{9'}$, -(C=O)-$NH_2$, -(C=O)-$NHR^{8'}$ or -(C=O)-$NR^{8'}R^{9'}$ whereby $R^{8'}$ and $R^{9'}$ for each substituent independently represent linear or branched $C_{1-6}$ alkyl,

$R^{5'}$ and $R^{6'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;

$R^{7'}$ represents hydrogen, a linear or branched $C_{1-6}$-alkyl group, a linear or branched $C_{1-6}$-alkoxy group, a halogen

atom, $CH_2F$, $CHF_2$, $CF_3$, CN, OH, $NO_2$, - (C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
and
(B) at least one lipid lowering compound used to treat dyslipidaemia.

Another aspect of the present invention relates to a active substance combination as decribed above, where the ingredient (A) is substituted pyrazoline of formula (IA) :

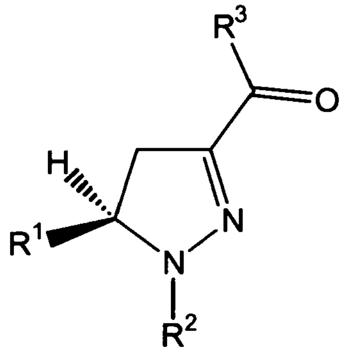

IA

wherein $R^1$, $R^2$, and $R^3$ are as defined above for formula (I).

A further aspect of the present invention relates to a active substance combination as decribed above, where the ingredient (A) is substituted pyrazoline of formula (IB):

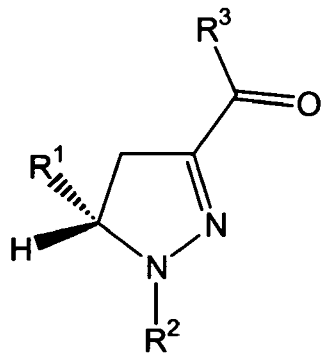

IB

wherein $R^1$, $R^2$, and $R^3$ are as defined above.

A further aspect of the present invention relates to a active substance combination as decribed above, where the ingredient (A) is substituted pyrazoline of formula (IC) :

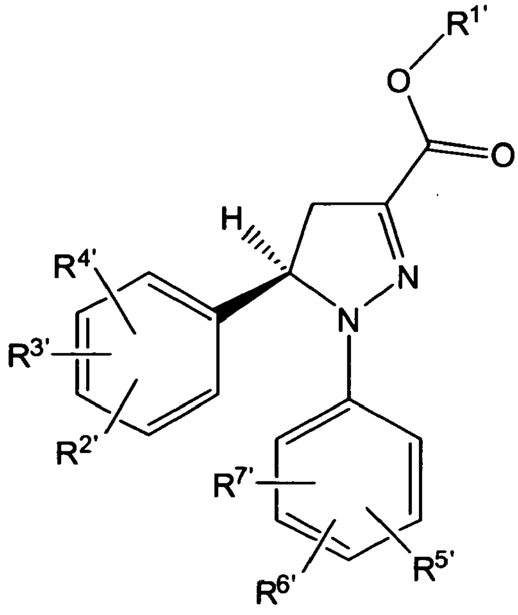

IC

wherein $R^{1'}$, $R^{2'}$, $R^{3'}$, $R^{4'}$, $R^{5'}$, $R^{6'}$, and $R^{7'}$ are as defined above.

A further aspect of the present invention relates to a active substance combination as decribed above, where the ingredient (A) is substituted pyrazoline of formula (ID) :

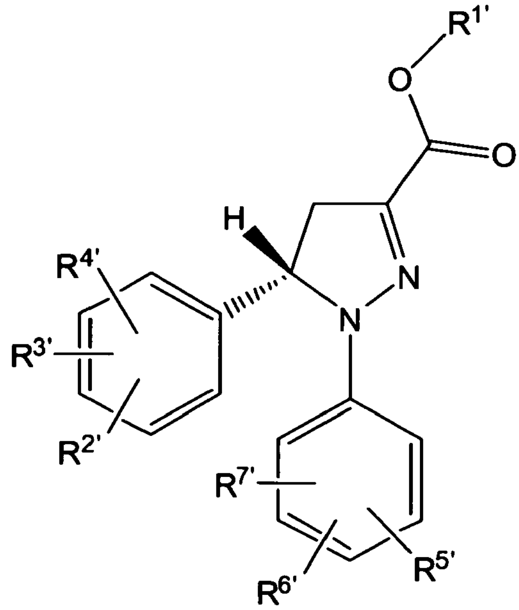

wherein R1', R2', R3', R4', R5', R6', and R7' are as defined above.

Preferably the active substance combination according to the present invention comprises one or more substituted pyrazoline compounds of general formula I given above, wherein $R^1$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R" whereby R' and R" for each substituent independently represent linear or branched $C_{1-6}$ alkyl, preferably $R^1$ represents a phenyl group, which is optionally

substituted by one or more substituents selected from the group consisting of methyl, ethyl, F, Cl, Br and $CF_3$, more preferably $R^1$ represents a phenyl group, which is mono-substituted with a chlorine atom in the 4-position.

Also, preferably the active substance combination according to the present invention comprises one or more substituted pyrazoline compounds of general formula I given above, wherein $R^2$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R", whereby R' and optionally R" for each substituent independently represent linear or branched $C_{1-6}$ alkyl, preferably $R^2$ represents a phenyl group, which is optionally substituted by one or more substituents independently selected from the group consisting of methyl, ethyl, F, Cl, Br and $CF_3$, more preferably $R^2$ represents a phenyl group, which is di-substituted with two chlorine atoms in its 2- and 4-position.

Also, preferably the active substance combination according to the present invention comprises one or more substituted pyrazoline compounds of general formula I given above, wherein $R^3$ represents a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or $R^3$ represents an optionally at least mono-substituted, 5- or 6-membered aryl or heteroaryl group, which may be condensed with an optionally at least mono-substituted mono- or polycyclic ring system, or $R^3$ represents an -$NR^4R^5$-moiety, preferably $R^3$ represents a saturated, optionally at least mono-substituted, optionally one or more nitrogen-atoms as ring member containing $C_{3-8}$ cycloaliphatic group, which may be condensed with an optionally at least mono-substituted mono- or polycyclic ring system, or $R^3$ represents an -$NR^4R^5$-moiety, more preferably $R^3$ represents a pyrrolidinyl group, a piperidinyl group or a piperazinyl group, whereby each of these groups may be substituted with one or more $C_{1-6}$-alkyl groups, or $R^3$ represents an -$NR^4R^5$-moiety.

Also, preferably the active substance combination according to the present invention comprises one or more substituted pyrazoline compounds of general formula I given above, wherein $R^4$ and $R^5$, identical or different, represent a hydrogen atom; an unbranched or branched, saturated or unsaturated, optionally at least mono-substituted $C_{1-6}$-aliphatic radical; a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system and/or bonded via a methylene (-$CH_2$-) or ethylene (-$CH_2$-$CH_2$)-group; an -$SO_2$-$R^6$-moiety; or an -$NR^7R^8$-moiety, preferably one of these residues $R^4$ and $R^5$ represents a hydrogen atom and the other one of these residues $R^4$ and $R^5$ represents a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system; an - $SO_2$-$R^6$-moiety; or an -$NR^7R^8$-moiety, or $R^4$ and $R^5$, identical or different, each represent a $C_{1-6}$ alkyl group, more preferably one of these residues $R^4$ and $R^5$ represents a hydrogen atom and the other one of these residues $R^4$ and $R^5$ represents an optionally at least mono-substituted pyrrolidinyl group; an optionally at least mono-substituted piperidinyl group; an optionally at least mono-substituted piperazinyl group; an optionally at least mono-substituted triazolyl group; an -$SO_2$-$R^6$-moiety; or an -$NR^7R^8$-moiety, or $R^4$ and $R^5$, identical or different, represent a methyl group, an ethyl group, an n-propyl group, an isopropyl group, an n-butyl group, a sec-butyl group or a tert.-butyl group.

Also, preferably the active substance combination according to the present invention comprises one or more substituted pyrazoline compounds of general formula I given above, wherein $R^6$ represents a linear or branched, saturated or unsaturated, optionally at least mono-substituted $C_{1-6}$ aliphatic group; a saturated or unsaturated, optionally at least mono-substituted, optionally at least one heteroatom as ring member containing $C_{3-8}$ cycloaliphatic group, which may be condensed with a mono- or polycyclic ring-system; or an optionally at least mono-substituted, 5- or 6-membered aryl or heteroaryl group, which may be condensed with a mono- or polycyclic ring system and/or bonded via a methylene (-$CH_2$-) or ethylene (-$CH_2$-$CH_2$)-group, preferably $R^6$ represents a $C_{1-6}$-alkyl group; a saturated, optionally at least mono-substituted cycloaliphatic group, which may be condensed with a mono- or polycyclic ring-system; or a phenyl group, which is optionally substituted with one or more $C_{1-6}$ alkyl groups.

Also, preferably the active substance combination according to the present invention comprises one or more substituted pyrazoline compounds of general formula I given above, wherein $R^7$ and $R^8$, identical or different, represent a hydrogen atom; an unbranched or branched, saturated or unsaturated, optionally at least mono-substituted $C_{1-6}$ aliphatic radical; a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system; or an optionally at least mono-substituted, 5- or 6 membered aryl or heteroaryl group, which may be condensed with an optionally at least mono-substituted mono- or polycyclic ring system and/or bonded via a methylene (-$CH_2$-) or ethylene (-$CH_2$-$CH_2$)-group, preferably $R^7$ and $R^8$, identical or different, represent a hydrogen

atom or a $C_{1-6}$ alkyl radical.

More preferably the active substance combination according to the present invention comprises one or more substituted pyrazoline compounds of general formula I given above, wherein

$R^1$ represents a phenyl group, which is optionally substituted with 1, 2, 3, 4 or 5 substituents independently selected from the group consisting of methyl, ethyl, F, Cl, Br and $CF_3$,

$R^2$ represents a phenyl group, which is optionally substituted with 1, 2, 3, 4 or 5 substituents independently selected from the group consisting of methyl, ethyl, F, Cl, Br and $CF_3$,

$R^3$ represents a pyrrolidinyl group, a piperidinyl group or a piperazinyl group, whereby each of these groups may be substituted with one or more of $C_{1-6}$-alkyl groups, or $R^3$ represents an $-NR^4R^5$-moiety,

$R^4$ represents a hydrogen atom or a linear or branched $C_{1-6}$-alkyl group,

$R^5$ represents a linear or branched $C_{1-6}$ alkyl group; an $-SO_2-R^6$-moiety; a pyrrolidinyl group; a piperidinyl group; a piperazinyl group; a homo-piperazinyl group; a morpholinyl group; a triazolyl group; whereby each of the heterocyclic rings may be substituted with one or more, identical or different, $C_{1-6}$-alkyl groups, and

$R^6$ represents a phenyl group, which is optionally substituted with one or more $C_{1-6}$ alkyl groups, which may be identical or different.

Also, more preferably the active substance combination according to the present invention comprises one or more substituted pyrazoline compounds of general formula I given above, wherein

$R^1$ represents a phenyl ring, which is mono-substituted with a halogen atom, preferably a chlorine atom, in its 4-position,

$R^2$ represents a phenyl ring, which is di-substituted with two halogen atoms, preferably chlorine atoms, in its 2- and 4-position,

$R^3$ represents a pyrrolidinyl group, a piperidinyl group, a piperazinyl group, a homo-piperazinyl group, a morpholinyl group, or an $-NR^4R^5$-moiety,

$R^4$ represents a hydrogen atom or a linear or branched $C_{1-6}$-alkyl group,

$R^5$ represents a linear or branched $C_{1-6}$ alkyl group; an $-SO_2-R^6$-moiety; a pyrrolidinyl group; a piperidinyl group; a piperazinyl group; a homo-piperazinyl group; a morpholinyl group; or a triazolyl group whereby each of the heterocyclic rings may be substituted with one or more, identical or different, $C_{1-6}$-alkyl groups, and

$R^6$ represents a phenyl group, which is optionally substituted with one or more $C_{1-6}$ alkyl groups, which may be identical or different.

Yet more preferably, the active substance combination according to the present invention comprises one or more substituted pyrazoline compounds of general formula I given above selected from the group consisting of

N-piperidinyl-5-(4-chloro-phenyl)-1-(2,4-dichlorophenyl)-4,5-dihydro-1H-pyrazol-3-carboxamide,

5-(4-Chloro-phenyl)-1-(2,4-dichloro-phenyl)-4,5-dihydro-1H-pyrazole-3-carboxylic acid-[1,2,4]-triazole-4-yl-amide,

5-(4-Chloro-phenyl)-1-(2,4-dichloro-phenyl)-4,5-dihydro-1H-pyrazole-3-carboxylic acid-(4-methyl-piperazin-1-yl)-amide,

5-(4-Chloro-phenyl)-1-(2,4-dichloro-phenyl)-4,5-dihydro-1H-pyrazole-3-carboxylic acid diethylamide,

[5-(4-Chloro-phenyl)-1-(2,4-dichloro-phenyl)-4,5-dihydro-1H-pyrazole-3-yl]-piperidine-1-yl-methanone and

N-[5-(4-Chloro-phenyl)-1-(2,4-dichlorophenyl)-4,5-dihydro-1H-pyrazole-3-carbonyl]-4-methylphenylsulfonamide,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also, more preferably the active substance combination according to the present invention may as the pyrazoline compound of general formula I comprise one of the following compounds

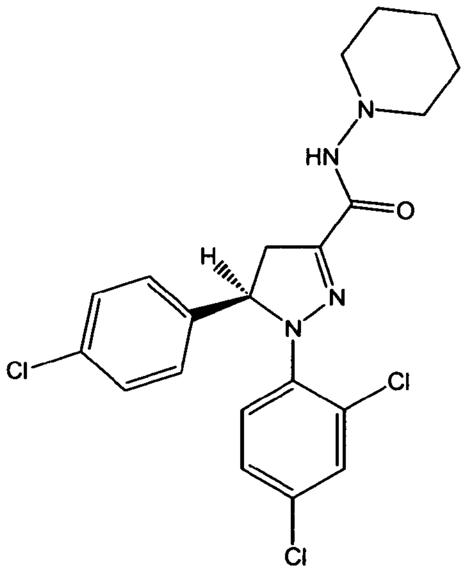 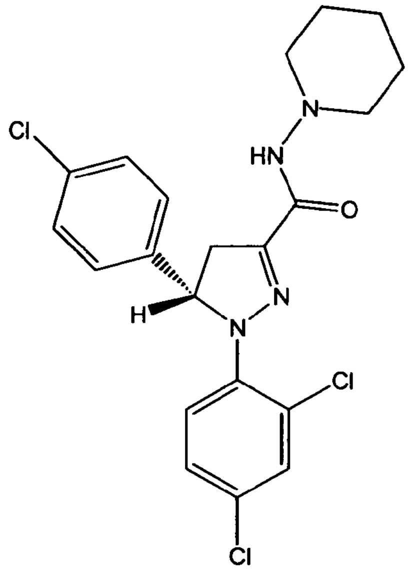

in each case optionally in the form of a corresponding N-oxide, a corresponding salt or a corresponding solvate thereof.

Also, preferably the active substance combination according to the present invention comprises one or more substituted pyrazoline compounds of general formula I' given above, wherein at least one of $R^{2'}$, $R^{3'}$ or $R^{4'}$ represents hydrogen, while at least one of $R^{2'}$, $R^{3'}$ or $R^{4'}$ is different from hydrogen.

Also, preferably the active substance combination according to the present invention comprises one or more substituted pyrazoline compounds of general formula I' given above, wherein $R^{7'}$ represents hydrogen.

Also, preferably the active substance combination according to the present invention comprises one or more substituted pyrazoline compounds of general formula I' given above, wherein $R^{2'}$, $R^{3'}$ and $R^{4'}$ independently of each other represent hydrogen, a linear or branched $C_{1-6}$-alkyl group, a halogen atom, or $CF_3$, preferably $R^{2'}$, $R^{3'}$ and $R^{4'}$ independently of each other represent hydrogen, methyl, ethyl, F, Cl, Br and $CF_3$.

Also, preferably the active substance combination according to the present invention comprises one or more substituted pyrazoline compounds of general formula I' given above, wherein $R^{5'}$ and $R^{6'}$ independently of each other represent a linear or branched $C_{1-6}$-alkyl group, a halogen atom, or $CF_3$, preferably $R^{5'}$ and $R^{6'}$ independently of each other represent methyl, ethyl, F, Cl, Br and $CF_3$.

Also, preferably the active substance combination according to the present invention comprises one or more substituted pyrazoline compounds of general formula I' given above, wherein $R^{2'}$ represents a chlorine atom in the 4-position of the phenyl ring, while $R^{3'}$ and $R^{4'}$ represent hydrogen.

Also, preferably the active substance combination according to the present invention comprises one or more substituted pyrazoline compounds of general formula I' given above, wherein $R^{5'}$ and $R^{6'}$ each represent a chlorine atoms in the 2- and 4-position of the phenyl ring, while $R^{7'}$ represents hydrogen.

Also, preferably the active substance combination according to the present invention comprises one or more substituted pyrazoline compounds of general formula I' given above, wherein $R^{1'}$ represents hydrogen, methyl or ethyl, preferably hydrogen.

Also, preferably the active substance combination according to the present invention comprises one or more substituted pyrazoline compounds of general formula I' given above, wherein the compounds of general formula I' are represented by the following structure II:

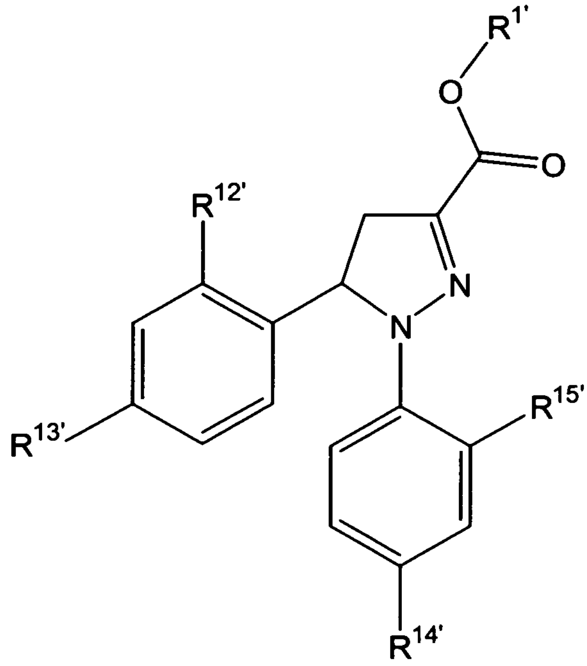

II

wherein
$R^{1'}$ represents hydrogen or a linear or branched $C_{1-4}$-alkyl group,
$R^{12'}$ or $R^{13'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$,
$R^{14'}$ or $R^{15'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, methyl, ethyl, F, Cl, Br and $CF_3$,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also, preferably the active substance combination according to the present invention comprises one or more substituted pyrazoline compounds of general formula I' given above, wherein in general structure II $R^{12'}$ and $R^{13'}$ independently of each other represent hydrogen, a linear or branched $C_{1-6}$-alkyl group, a halogen atom, or $CF_3$, preferably $R^{12'}$ and $R^{13'}$ independently of each other represent hydrogen, methyl, ethyl, F, Cl, Br and $CF_3$.

Also, preferably the active substance combination according to the present invention comprises one or more substituted pyrazoline compounds of general formula I' given above, wherein in general structure II $R^{14'}$ and $R^{15'}$ independently of each other represent a linear or branched $C_{1-6}$-alkyl group, a halogen atom, or $CF_3$, preferably $R^{14'}$ and $R^{15'}$ independently of each other represent methyl, ethyl, F, Cl, Br and $CF_3$.

Also, preferably the active substance combination according to the present invention comprises one or more substituted pyrazoline compounds of general formula I' given above, wherein in general structure II $R^{13'}$ represents Cl and $R^{12'}$ represents hydrogen.

Also, preferably the active substance combination according to the present invention comprises one or more substituted pyrazoline compounds of general formula I' given above, wherein in general structure II $R^{14'}$ and $R^{15'}$ each represent Cl.

Also, preferably the active substance combination according to the present invention comprises one or more substituted pyrazoline compounds of general formula I' given above, wherein in general structure II $R^{1'}$ represents hydrogen, methyl or ethyl, preferably hydrogen.

Also, preferably the active substance combination according to the present invention comprises the compound 5-(4-chloro-phenyl)-1-(2,4-dichlorophenyl)-4,5-dihydro-1H-pyrazol-3-carboxylic acid,
optionally in form of one of the stereoisomers, preferably enantiomers or diastereomers, a racemate or in form of a mixture of at least two of the stereoisomers, preferably enantiomers and/or diastereomers, in any mixing ratio, or a

corresponding salt thereof, or a corresponding solvate thereof as compound of general formula I'.

Also, more preferably the active substance combination according to the present invention may as the pyrazoline compound of general formula I' comprise one of the following compounds

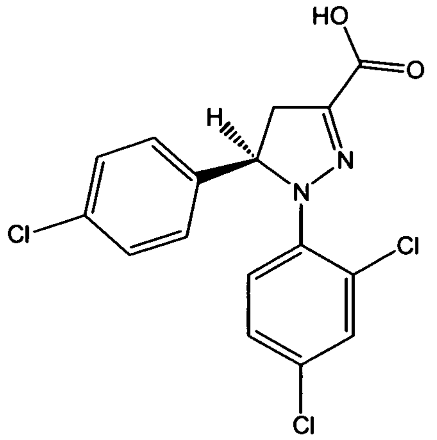

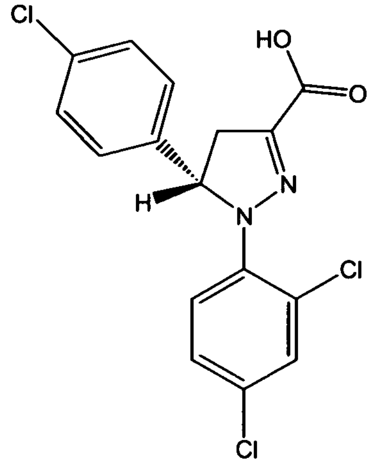

in each case optionally in the form of a corresponding N-oxide, a corresponding salt or a corresponding solvate thereof.

The other active component of the inventive active substance combination, component (B), is a lipid lowering compound, used to treat dyslipidaemia.

The term "lipid lowering compound" as used herein includes all types of drugs used to treat dyslipidaemia.

In a broad aspect, drugs to treat dyslipidaemia are defined as those which rectify plasma lipid abnormalities that are known to be harmful to health or well being. These drugs reduce excessive plasma concentrations of triglycerides, cholesterol, its low density esters, eg VLDL-cholesterol, LDL-cholesterol, IDL-cholesterol, and/or apo-lipoprotein-B and/or increase the concentrations of the so-called "good cholesterol" ie high density cholesterol (HDL-cholesterol).

More particularly for the purpose of the present invention, lipid lowering compounds, used to treat dyslipidaemia include, but are not limited to, the following pharmacological classes, viz 3-hydroxy-3-methylglutaryl coenzyme A (HMG-CoA) reductase inhibitors (statins), the nuclear peroxisome proliferator activated receptor-$\alpha$ (PPAR$\alpha$) agonists (fibrates), bile acid sequestrants, cholesterol absorption blockers, niacin preparations, cholesteryl ester transfer protein (CETP) inhibitors and combinations thereof.

Lipid-lowering compounds as encompassed by the present invention, both marketed and/or in development, include, but are not limited to, at least 1 selected from the following non-limiting list of examples: fibrates, (eg bezafibrate, cholestyramine, ciprofibrate, clofibrate, colestipol, fenofibrate, gemfibrozil), the statins (eg 4"-hydroxymevastatin lactone, atorvastatin, atorvastatin calcium, bervastatin, carvastatin, cerivastatin, compactin, crilvastatin, dalvastatin, dihydrocompactin, epastatin, epistatin, eptastatin sodium, estatin A, fluindostatin, fluindostatin sodium, fluvastatin, glenvastatin, isosimvastatin-6-one, itavastatin, lipstatin, lovastatin, mevastatin, mevinolin, monakolin K, mumbaistatin, nisvastatin, NO-pravastatin, prevastatin, pitavastatin, plavastatin, pravastatin, rivastatin, rosuvastatin, simvastatin, squalestatin, synvinolin, tetrahydrolipstatin, velostatin, zaragozic acid A), bile acid sequestrants (eg colsevelam, cholestyramine, colestipol), cholesterol absorption blockers (eg ezetimibe, avasimibe), nicotinic acid (or niacin, including niaspan), or other compounds to treat dyslipidaemia that are either in research or in development including, but not limited to, cholesteryl ester transfer protein (CETP) inhibitors (eg torcetrapib, JJT-705), microsomal triglyceride transfer protein inhibitors (eg implitapide), omacor, squalane synthase inhibitors (eg schizostatin), sterol-regulatory element binding protein (SREBP) cleavage-activating protein (SCAP) ligands, ribA-87049, AF-15831, AR-121, aSS-28, AY-27773, Bay-w-6228, BIM-23014C, BIM-23027, BIM-23052, BIM-23056, BIM-23197, BIM-23206, BIM-23268, BIM-23268D, BIM-23454, BIM-23627, BN-52030, BN-82176, BW-B633C, CETi-1, F-10463A, HR-780, ICI-213878, ITM-014, KB-3305, CS-500, CS-514, CS-866PRV, CI-981, DC13-116, DC-23-99, INS-1, KS-01-018, KS-01-019, L-054264, L-054522, L-054852, L-154803, L-166143, L-362855, L49-vcMMAF, L-637312, L-642957, L-669262, L-796778, L-797591, L-803087, L-817818, E-64-d, EN-100, ES-305, ES-6864, ES-8891, EST, LS-2904, MK-733, MK-791, MK-803, ML-236B, MND-21, NCX-6550, NCX-6553, NCX-6554, NK-104, NKS-104, MT5-hAST, NR-300s, P 23924A, PRL-2894, R-212, RG-12561, PMD-387, Ro-090154, Ro-18-0647, Ro-18-0647/002, RS-8891, S-4522, S-8921, SB-710411, SDZ-221-047ac, SQ-31000, SR 42654, SR 42991, SR 43062, SR-43571, SR-43845, SRI 62320, TF-802, U-70504E, WOC-3B, XU-620, XU-62-320, YH-137, YH-138, YM-548 and ZD-4522. Compounds selected from the above non-limiting list may be used alone or in

combination together with 1 or more substituted pyrazolines according to the present invention, in the said combination medicament.

Those skilled in the art will recognize that some of the above statins contain either a free carboxlic acid or a free amine group as part of their chemical structure. Some statins contain lactone moieties, which exist in quilibrium with the free carboxylic acid form. Such lactones can be maintained as carboxylates by preparing pharmaceutically active salts of the lactone. Such salts are also encompassed by the present invention.

Preferably, the statin compound of component (B) may be selected from the group consisting of atorvastatin, rosuvastatin, simvastatin, pravastatin, cerivastatin, mevastatin, velostatin, fluvastatin, compactin, dihydrocompactin, lovastatin, dalvastatin and fluindostatin, optionally in the form of a corresponding salt or a corresponding solvate thereof.

More preferably, the statin compound of component (B) may be selected from the group consisting of selected from the group consisting of atorvastatin, atorvastatin-calcium, rosuvastatin, rosuvastatin-sodium, simvastatin, pravastatin, pravastatin-sodium, cerivastatin, cerivastatin-sodium, mevastatin, velostatin, fluvastatin, fluvastatin-sodium, compactin, dihydrocompactin, lovastatin, dalvastatin and fluindostatin.

The statins may be prepared by methods well known to those skilled in the art. In particular, simvastatin may be prepared by the method disclosed in US 4,444,784, pravastatin may be prepared by the method disclosed in US 4,346,227, cerivastatin may be prepared by the method disclosed in US 5,502,199, EP617019 or EP0325130, mevastatin may be prepared by the method disclosed in US 3,983,140, velostatin may be prepared by the method disclosed in US 4,448,784 and US 4,450,171, fluvastatin may be prepared by the method disclosed in US 4,739,073 or EP0244364, compactin may be prepared by the method disclosed in US 4,804,770, lovastatin may be prepared by the method disclosed in US 4,231,938 or EP0306210, dalvastatin may be prepared by the method disclosed in EP 738510, fluindostatin may be prepared by the method disclosed in EP 363934, atorvastatin may be prepared by the method disclosed in US 4,681,893, atorvastatin-calcium may be prepared by the method disclosed in US 5,273,995, Rosuvastatin may be prepared by the method disclosed in US 5,260,440 and dihydrocompatin may be prepared by the method disclosed in US 4,450,171. The respective parts of the descriptions are hereby incorporated by reference and form part of the present disclosure.

Several substituted pyrazolines according to the present invention have been demonstrated to have beneficial effects on obesity, visceral adiposity, appetency disorders, insulin resistance/impaired glucose tolerance [pre-diabetes], Type 2 diabetes, Metabolic Syndrome and drug-induced weight-gain/obesity. A medicament comprising at least 1 compound selected from substituted pyrazolines according to the present invention, together with at least 1 lipid-lowering agent from the non-limiting list given above shows synergistics benefits to patients suffering from dyslipidaemia because the actions of the CB1 receptor antagonist not only acts directly to improve the lipid profiles of patients, but its actions reduce other causative factors of dyslipidaemia which is of exceptional therapeutic assistance. This broad spectrum of improvement provides additional clinical benefit to a patient with dyslipidaemia by preventing or ameliorating deleterious changes in food-related, cardio-metabolic risk factors, ie obesity, increased visceral adiposity and/or increases in plasma triglycerides, cholesterol and/or LDL-cholesterol, decreases in HDL-cholesterol, insulin resistance and/or impaired glucose tolerance and/or hypertension that predisposes him/her to a greatly increased risk of developing Type 2 diabetes and/or Metabolic Syndrome, or of suffering a cardio- or cerebrovascular accident.

A combination medicament comprising at least 1 substituted pyrazoline according to the present invention, as defined above, which is a CB1 receptor antagonist or inverse agonist, together with an at least one of lipid lowering agent usefull to treat dyslipidaemia and selected from the non-limiting list of compounds given above has therapeutic benefits in the treatment of dyslipidaemia and/or food-related disorders.

The actions of the said combination medicament demonstrate to be synergistic because this combination medicament produces a greater improvement in abnormal plasma lipid profiles than the lipid lowering compound (or compounds) used to treat dyslipidaemia when given alone.

Further said combination medicament reduces craving for and the excessive consumption of palatable high-fat foods that exacerbate dyslipidaemia and are a major cause of dyslipidaemia and dyslipidaemia-related metabolicdisorders.

In addition, the presence of a substituted pyrazoline as defined above, in the said combination delivers weight-loss-independent decreases in plasma triglycerides and increases in HDL-cholesterol (the so-called "good cholesterol") and improvements glycaemic control [improving dyslipidaemia by preventing the progression of insulin resistance/impaired glucose tolerance (pre-diabetes) to Type 2 diabetes or by reducing the contribution to the disease from pre-existing Type 2 diabetes].

Finally as described above, the presence of a substituted pyrazoline according to the present invention, in the said combination will delivers benefits, through weight-loss and actions on macronutrient choices, on obesity, visceral adiposity, appetency disorders, insulin resistance/impaired glucose tolerance [pre-diabetes], Type 2 diabetes, Metabolic Syndrome and drug-induced weight-gain/obesity.

In summary, a medicament comprising at least 1 compound selected from substituted pyrazolines as described above, together with at least lipid lowering compound exhibit synergistic benefits in ameliorating dyslipidaemia in non-obese subjects who are suffering only from dyslipidaemia, or dyslipidaemia in combination with insulin resistance and/or impaired glucose tolerance and/or Type 2 diabetes and/or Metabolic Syndrome because of its weight-loss independent beneficial actions on plasma lipids and glycaemic control. The said combination is also synergistically efficacious in overweight or obese patients with dyslipidaemia when it is associated with other cardio-metabolic risk factors by virtue of its weight-loss-independent effects on plasma lipids and glycaemic control, and in addition, through its actions to reduce obesity, visceral adiposity, plasma triglycerides, increase HDL-cholesterol, and/or reduce hypertension. A substituted pyrazoline according to the present invention in the said combination also reduces cravings for the consumption of high fat foods thereby improving dyslipidaemia. Finally, a substituted pyrazoline according to the present invention in the said combination prevents or reduces drug-induced weight-gain or obesity that will exacerbate pre-existing dyslipidaemia and is also a major cause of dyslipidaemia. Thus, in all of these patient groups, a substituted pyrazoline according to the invention and described above, in combination with at least one lipid lowering compound used to treat dyslipidaemia provides synergistic clinical benefit by preventing or ameliorating deleterious changes in food-related, cardio-metabolic risk factors, ie that predispose the patient to a greatly increased risk of developing dyslipidaemia and/or insulin resistance and/or impaired glucose tolerance and/or Type 2 diabetes and/or Metabolic Syndrome, or of suffering a cardio- or cerebrovascular accident.

More preferred is an inventive active substance combination comprising

(A) at least one substituted pyrazoline compound of general formula I

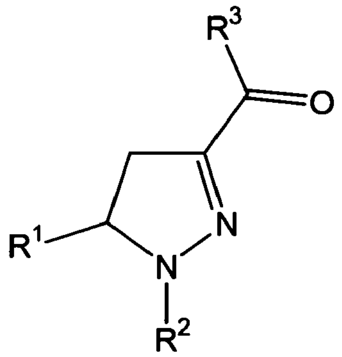

I

wherein

$R^1$ represents a phenyl group, which is optionally substituted with 1, 2, 3, 4 or 5 substituents independently selected from the group consisting of methyl, ethyl, F, Cl, Br and $CF_3$,

$R^2$ represents a phenyl group, which is optionally substituted with 1, 2, 3, 4 or 5 substituents independently selected from the group consisting of methyl, ethyl, F, Cl, Br and $CF_3$,

$R^3$ represents a pyrrolidinyl group, a piperidinyl group or a piperazinyl group, whereby each of these groups may be substituted with one or more of $C_{1-6}$-alkyl groups, or $R^3$ represents an $-NR^4R^5$-moiety,

$R^4$ represents a hydrogen atom or a linear or branched $C_{1-6}$-alkyl group,

$R^5$ represents a linear or branched $C_{1-6}$ alkyl group; an $-SO_2-R^6$-moiety; a pyrrolidinyl group; a piperidinyl group; a piperazinyl group; a homo-piperazinyl group; a morpholinyl group; a triazolyl group; whereby each of the heterocyclic rings may be substituted with one or more, identical or different, $C_{1-6}$-alkyl groups, and

$R^6$ represents a phenyl group, which is optionally substituted with one or more $C_{1-6}$ alkyl groups, which may be identical or different.

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,

and/or

at least one substituted pyrazoline compound of general formula I' represented by the following structure II:

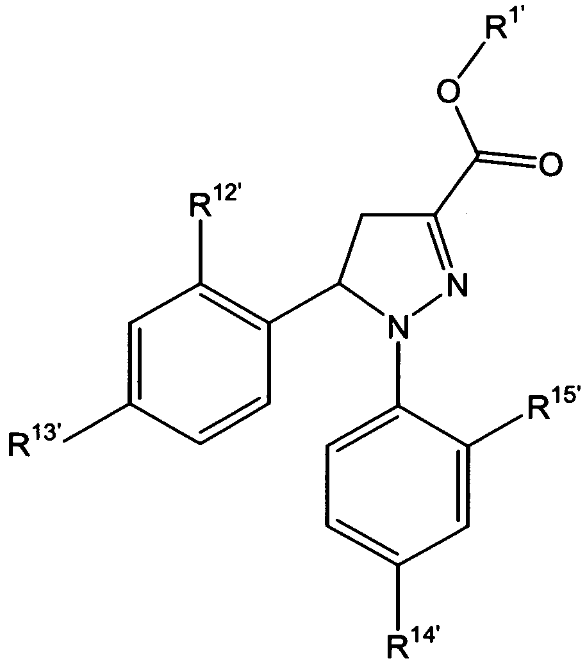

II

wherein
$R^{1'}$ represents hydrogen or a linear or branched $C_{1-4}$-alkyl group,
$R^{12'}$ or $R^{13'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$,
$R^{14'}$ or $R^{15'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, methyl, ethyl, F, Cl, Br and $CF_3$,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
and

(B) at least one statin compound selected from the group consisting of atorvastatin, rosuvastatin, simvastatin, pravastatin, cerivastatin, mevastatin, velostatin, fluvastatin, compactin, dihydrocompactin, lovastatin, dalvastatin and fluindostatin, optionally in the form of a corresponding salt or a corresponding solvate thereof.

Yet more preferred is an inventive active substance combination comprising
one or more substituted pyrazoline compounds of general formula I selected from the group consisting of
N-piperidinyl-5-(4-chloro-phenylyl-(2,4-dichlorophenyl)-4,5-dihydro-1H-pyrazol-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone and
N-[5-(4-Chloro-phenyl)-1-(2,4-dichlorophenyl)-4,5-dihydro-1H-pyrazole-3-carbonyl]-4-methylphenylsulfonamide,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,
and/or
5-(4-chloro-phenyl)-1-(2,4-dichlorophenyl)-4,5-dihydro-1H-pyrazol-3-carboxylic acid
optionally in form of one of the stereoisomers, preferably enantiomers or diastereomers, a racemate or in form of a

mixture of at least two of the stereoisomers, preferably enantiomers and/or diastereomers, in any mixing ratio, or a corresponding salt thereof, or a corresponding solvate thereof,
and

(B) at least one statin compound selected from the group consisting of atorvastatin, rosuvastatin, simvastatin, pravastatin, cerivastatin, mevastatin, velostatin, fluvastatin, compactin, dihydrocompactin, lovastatin, dalvastatin and fluindostatin, optionally in the form of a corresponding salt or a corresponding solvate thereof.

The inventively used substituted pyrazoline compounds may, for example, be obtained by the following process, according to which which at least one benzaldehyde compound of general formula II

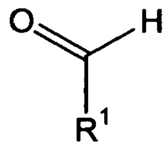

**(II)**

wherein $R^1$ has the meaning given above, is reacted with a pyruvate compound of general formula (III)

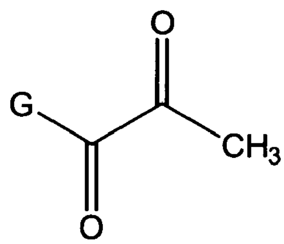

**(III),**

wherein G represents an OR group with R being a branched or unbranched $C_{1-6}$ alkyl radical, preferably an ethyl radical, or G represents an O-K group with K being a cation, preferably a monovalent cation, more preferably an alkali metal cation, even more preferably a sodium cation, to yield a compound of general
formula (IV)

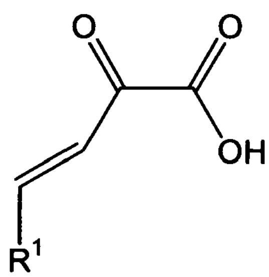

(IV)

wherein $R^1$ has the meaning given above, which is optionally isolated and/or optionally purified, and which is reacted with an optionally substituted phenyl hydrazine of general formula (V)

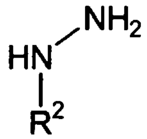

(V)

or a corresponding salt thereof, wherein $R^2$ has the meaning given above, under an inert atmosphere, to yield a compound of general formula (VI)

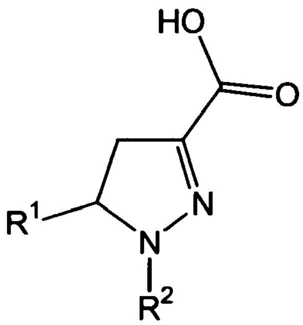

(VI)

wherein $R^1$ and $R^2$ have the meaning as given above, which is optionally isolated and/or optionally purified, and optionally transferred under inert atmosphere to a compound of general formula (VII)

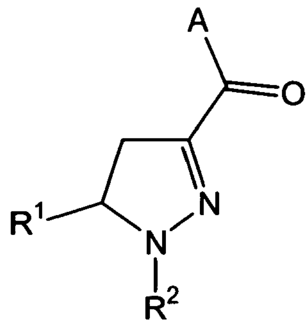

(VII)

wherein the substituents $R^1$ and $R^2$ have the meaning given above and A represents a leaving group, via the reaction with an activating agent, said compound being optionally isolated and/or optionally purified, and at least one compound of general formula (VI) is reacted with a compound of general formula $R^3H$, wherein $R^3$ represents an $-NR^4R^5$-moiety, wherein $R^4$ and $R^5$ have the meaning given above, to yield a substituted pyrazoline compound of general formula I, wherein $R^3$ represents an $-NR^4R^5$-moiety,
and/or at least one compound of general formula (VII) is reacted with a compound of the general formula $R^3H$, in which $R^3$ has the meaning given above to yield a compound of general formula (I) given above, which is optionally isolated and/or optionally purified.

The process is also illustrated in scheme I given below:

**Scheme I:**

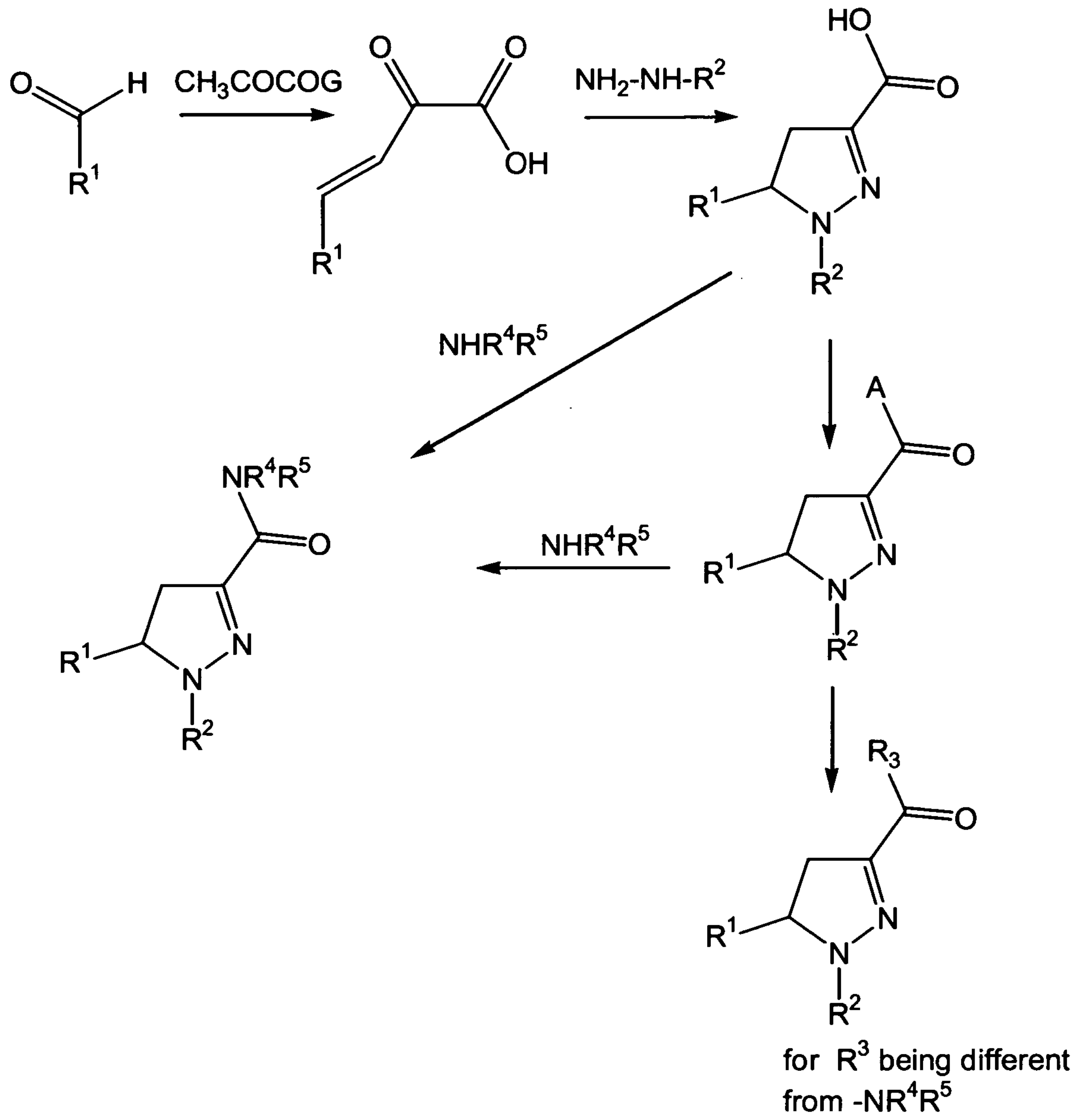


The reaction of the benzaldehyde compound of general formula II with a pyruvate compound of general formula III is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably sodium pyruvate may be used as the pyruvate compound. Preferably said reaction is carried out in a protic reaction medium such as a $C_{1-4}$ alkyl alcohol or mixtures of these. Mixtures of such alcohols with water, e.g. ethanol/water may also be used.

Reaction temperature as well as the duration of the reaction may vary over a broad range. Preferred reaction temperatures range from -10 °C to the boiling point of the reaction medium. Suitable reaction times may vary for example from several minutes to several hours.

Also preferred the reaction of the benzaldehyde compound of general formula II with a pyruvate compound of general formula III is carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of general formula (IV) with an optionally substituted phenyl hydrazin of general formula (V) is preferably carried out in a suitable reaction medium such as $C_{1-4}$-alcohols or ethers such as dioxane or tetrahydrofurane or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of general formula (VI) may be activated for further reactions by the introduction of a suitable leaving group according to conventional methods well known to those skilled in the art. Preferably the compounds of general formula (VI) are transferred into an acid chloride, an acid anhydride, a mixed anhydride, a $C_{1-4}$ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (VII) is an acid chloride, it is preferably prepared by reaction of the corresponding acid of general formula (VI) with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0° C to the boiling point of the solvent and reaction times from several minutes to several hours.

If said activated compound of general formula (VII) is a mixed anhydride, said anhydride may preferably be prepared, for example, by reaction of the corresponding acid of general formula (VI) with ethyl chloroformiate in the presence of a base such as triethylamine or pyridine, in a suitable solvent.

The reaction of general formula (VII) with a compound of general formula $HR^3$ to yield compounds of general general I, wherein $R^3$ represents an - $NR^4R^5$ moiety is preferably carried out in presence of a base such as triethylamine in a reaction medium such as methylenchloride. The temperature is preferably in the range from 0°C to the boiling point of the reaction medium. The reaction time may vary over a broad range, e.g. from several hours to several days.

The reaction of general formula (VII) with a compound of general formula $HR^3$ to yield compounds of general formula I, wherein $R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system may be carried out according to conventional methods well known to those skilled in the art, e.g. from Pascual, A., J. Prakt Chem., 1999, 341 (7), 695-700; Lin, S. et al., Heterocycles, 2001, 55(2), 265-277; Rao, P. et al., J. Org. Chem., 2000, 65(22), 7323-7344, Pearson D.E and Buehler, C.A., Synthesis, 1972, 533-542 and references cited therein. The respective descriptions are hereby incorporated by reference and form part of the present disclosure.

Preferably said reaction is carried out in the presence of a Lewis acid, which is preferably selected from the group consisting of $FeCl_3$, $ZnCl_2$ and $AlCl_3$, in a suitable reaction medium such as toluene, benzene, tetrahydrofurane or similar. The temperature is preferably in teh range from 0°C to the boiling point of the reaction medium, more preferably from 15 to 25 °C. The reaction time may vary over a broad range, e.g. from several minutes to several hours.

The afore mentioned reactions involving the synthesis of the 4,5-dihydro-pyrazole ring or the reaction of a compound comprising said ring are carried out under an inert atmosphere, preferably nitrogen or argon, to avoid oxidation of the ring-system.

During the processes described above the protection of sensitive groups or of reagents may be necessary and/or desirable. The introduction of conventional protective groups as well as their removal may be performed by methods well-known to those skilled in the art.

If the substituted pyrazoline compounds of general formula (I) themselves are obtained in form of a mixture of stereoisomers, particularly enantiomers or diastereomers, said mixtures may be separated by standard procedures known to those skilled in the art, e.g. chromatographic methods or fractunalized crystallization with chiral reagents. It is also possible to obtain pure stereoisomers via stereoselective synthesis.

Salts of the inventively used active compounds may be obtained by a process, wherein at least one of the compounds having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid,

methanesulfonic acid or camphersulfonic acid.

Salts of the inventively used active compounds may also be prepared by a method, wherein at least one of the compounds having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. $[NH_nR_{4-n}]^+$, wherein n is 0, 1, 2, 3 or 4 and R represents a branched or unbranched $C_{1-4}$-alkyl-radical. Suitable reaction media are, for example, any of the ones given above.

Solvates, preferably hydrates, of the inventively used substituted pyrazoline compounds of general formula (I), of corresponding stereoisomers, of corresponding N-oxides or of corresponding salts thereof may also be obtained by standard procedures known to those skilled in the art.

Substituted pyrazoline compounds of general formula I, which comprise nitrogenatom containing saturated, unsaturated or aromatic rings may also be obtained in the form of their N-oxides by methods well known to those skilled in the art and used in the active substance combination of the present invention.

Those skilled in the art understand that the term substituted pyrazoline compounds as used herein is to be understood as encompassing derivatives such as ethers, esters and complexes of these compounds as well. The term "derivatives" as used in this application is defined here as meaning a chemical compound having undergone a chemical derivation starting from an acting (active) compound to change (ameliorate for pharmaceutical use) any of its physico-chemical properties, especially a so-called prodrug, e.g. their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002). The respective description is hereby incorporated by reference and forms part of the disclosure.

The purification and isolation of the inventively used substituted pyrazoline compounds of general formula (I), of a corresponding stereoisomer, or salt, or N-oxide, or solvate or any intermediate thereof may, if required, be carried out by conventional methods known to those skilled in the art, e.g. chromatographic methods or recrystallization.

The inventive active substance combination may comprise the components (A) and (B) in a molar ratio of component (A) to component (B) in the range of 1:10 to 10:1, preferably 1:5 to 5:1.

The inventive active substance combination is suitable for the administration to humans, including infants, children and grown-ups, as well as animals.

Preferably the total amount of the active substance(s) according to component (A), calculated as the free compound(s), to be administered to the patient in a 24 hours period does not exceed 800 mg, preferably does not exceed 500 mg.

The total amount of the active substance(s) according to component (B), calculated as the free compound(s), to be administered to the patient in a 24 hours period does preferably not exceed 160 mg, more preferably does not exceed 80 mg.

Preferably the inventive active substance combination comprises components (A) and (B) in the above defined molar ratios and within the afore given limits for the maximum dosis to be administered per day. Medicaments on the basis of the inventive active substance combination may preferably be administered once daily, twice daily or three times daily, more preferably once daily or twice daily, most preferably once daily.

In another aspect the present invention relates to a medicament comprising an inventive active substance combination according to the present invention and for which (B) is not a statin, and optionally at least one further active substance and/or optionally at least one auxiliary substance.

Particularly preferred is the use of an active substance combination of (A) and (B) according to the present invention and optionally one or more pharmaceutically acceptable excipients, for the preparation of a medicament for the treatment of dyslipidaemia.

More particularly the active substance combination of (A) and (B) according to the present invention and optionally one or more pharmaceutically acceptable excipients, is useful for the preparation of a medicament for the treatment of and/or obesity, visceral adiposity and/or appetency disorders and/or pre-diabetes (insulin resistance, impaired glucose tolerance) or Type 2 diabetes and/or Metabolic Syndrome, including drug-induced weight-gain or drug-induced obesity.

Another particular aspect of the invention concerns the use of an active substance combination of (A) and (B) according to the present invention and optionally one or more pharmaceutically acceptable excipients, for reducing the excessive consumption of palatable high-fat foods, both sweet (ground milk and chocolate) and savoury (ground roasted salted peanuts), as well as those contaning high proportion of saturated fats.

A further aspect of the invention relates to the use of an active substance combination of (A) and (B) according to the present invention and optionally one or more pharmaceutically acceptable excipients, for the preparation of a medicament for reducing drug induced weight gain or drug indiced obesity.

Specifically, the inventive medicament is suitable for the modulation of cannabinoid-receptors, preferably cannabinoid 1 ($CB_1$) receptors; and/or for HMG-CoA reductase inhibition.

In another aspect of the invention, the inventive medicament containing an active substance combination of (A) and (B) according to the present invention, and for which (B) is not a statin, is useful for the prophylaxis and/or treatment of coronary heart disease, myocardial ischemia, angina pectoris, atherosclerosis, hypertension, hyperlipidemia, Lipoprotein disorders, Hypercholesterolemia, or for lowering of cardiac risk.

Cardiac risk as used herein means that a subject (human or animal) will suffer a future adverse cardiac event such as, e.g. myocardial infarction, cardiac arrest, cardiac failure, cardiac ischemia. Cardiac risk can be calculated using the Framingham Risk equation, as disclosed with respect to the Framingham Heart Study, in Wilson et al., Am. J. Cardiol. 1987, 59(14):91G-94G. The respective part of the literature is herewith incorporated by reference and forms part of the present disclosure.

The inventive medicament according to this aspect of the invention, containing an active substance combination of (A) and (B) according to the present invention, and for which (B) is not a statin, is also suitable for the modulation of cannabinoid-receptors, preferably cannabinoid 1 ($CB_1$) receptors; for the regulation of triglyceride levels in the blood plasma; for the prophylaxis and/or treatment of disorders of the central nervous system; for the prophylaxis and/or treatment of disorders of the cardiovascular system; for the prophylaxis and/or treatment of disorders of the immune system; for the prophylaxis and/or treatment of disorders of the endocrinous system; for the prophylaxis and/or treatment of disorders of the respiratory system; for the prophylaxis and/or treatment of disorders of the gastrointestinal tract and/or for the prophylaxis and/or treatment of reproductive disorders.

Another aspect of the invention is the use of an active substance combination of (A) and (B) according to the present invention, and for which (B) is not a statin for the manufacture of a medicament for improvent of cardiovascular and/or metabolic risk factors, such as one or more of the following factors:

Elevated triglycerides, whereby elevated levels of triglycerides are preferably understood as being > 150 mg/dl,

Low HDL cholesterol, whereby low levels of HDL cholesterol are preferably understood as being < 40 mg/dl in men and < 50 mg/dl in women,

Hypertension, whereby hypertension is preferably understood as being > 130/85 mmHg,

Impaired fasting glucose, whereby impaired fasting glucose levels are preferably understood as being > 110 mg/dl,

Insulin resistance.

Another aspect of the invention is the use of an active substance combination of (A) and (B) according to the present invention, and for which (B) is not a statin for the manufacture of a medicament for the treatment of the weight independent aspects of metabolic syndrome.

Another aspect of the invention is a method for improving cardiovascular and/or metabolic risk factors, such as one or more of the following factors:

Elevated triglycerides, whereby elevated levels of triglycerides are preferably understood as being > 150 mg/dl,

Low HDL cholesterol, whereby low levels of HDL cholesterol are preferably understood as being < 40 mg/dl in men and < 50 mg/dl in women,

Hypertension, whereby hypertension is preferably understood as being > 130/85 mmHg,

Impaired fasting glucose, whereby impaired fasting glucose levels are preferably understood as being > 110 mg/dl,

Insulin resistance

Dyslipidemia,

in a subject, preferably a human.

Another aspect of the invention is a method for treating of the weight independent aspects of metabolic syndrome.

Since the pyrazoline compounds as defined herein also have a beneficial effect on the ratio of low density lipoprotein (LDL) to high density lipoprotein (HDL), i.e. they lower the LDL-levels and/or elevate the HDL levels; they are also useful as coating material or co-coating material in stents in order to prevent restenosis.

Moreover, the inventive medicament containing an active substance combination of (A) and (B) according to the present invention, and for which (B) is not a statin, is also suitable for the prophylaxis and/or -treatment of one or more of the following disorders:

food intake disorders, preferably selected from the group consisting of bulimia; anorexia; cachexia; psychosis; alcohol abuse and/or addiction; nicotine abuse and/or addiction; drug abuse and/or addiction; medicament abuse and/or addiction; schizophrenia; anxiety; depression; epilepsy; neurodegenerative disorders, preferably Morbus Parkinson; Morbus Huntington; Morbus Alzheimer and/or Multiple Sclerosis; cerebellar disorders; spinocerebellar disorders; cognitive disorders; cranial trauma; panic attacks; peripheric neuropathy; glaucoma; migraine; Raynaud's disease; tremblement disorders; compulsive disorders; senile dementia; thymic disorders; tardive dyskinesia; bipolar disorders; bone disorders including osteoporosis or Paget's disease of bone; cancer, preferably for the prophylaxis and/or treatment of one or more types of cancer selected from the group consisting of brain cancer; bone cancer; lip cancer; mouth cancer; esophageal cancer; stomach cancer; liver cancer; bladder cancer; pancreas cancer; ovary cancer; cervical cancer; lung cancer; breast cancer; skin camcer; colon cancer; bowl cancer and prostate cancer, more preferably for the prophylaxis and/or treatment of one or more types of cancer selected from the group consisting of colcon cancer; bowel cancer and prostate cancer, medicament-induced movement disorders; dystonia; endotoxemic shock; stroke; hemorragic shock; hypotension; insomnia; immunologic disorders; sclerotic plaques; vomiting; diarrhea; asthma; memory disorders; pruritus; pain; or for potentiation of the analgesic effect of narcotic and non-narcotic analgesics; or for influencing intestinal transit.

Those skilled in the art understand that the components (A) and (B) of the active substance combination as well as the different components of components (A) may be administered simultaneously or sequentially to one another, whereby in each case components (A) (including one or both of substituted pyrazoline compounds of general formula I and I') and (B) may be administerd via the same or different administration pathways, e.g. orally or parenterally. preferably both components (A) and (B) are administered simultaneously in one and the same administration form.

Another aspect of the present invention relates to the use of an inventive pharmacologically active substance combination according to the present invention, and for which (B) is not a statin, for the preparation of a medicament for the modulation of cannabinoid-receptors, preferably cannabinoid 1 ($CB_1$) receptors; and/or for HMG-CoA reductase inhibition.

Another aspect of the present invention relates to the use of an inventive pharmacologically active substance combination according to the present invention, and for which (B) is not a statin, for the preparation of a medicament for the prophylaxis and/or treatment of coronary heart disease, myocardial ischemia, angina pectoris, atherosclerosis, hypertension, hyperlipidemia, Lipoprotein disorders, Hypercholesterolemia, or for lowering of cardiac risk.

Another aspect of the present invention relates to the use of an inventive pharmacologically active substance combination according to the present invention, and for which (B) is not a statin, for the preparation of a medicament for the modulation of cannabinoid-receptors, preferably cannabinoid 1 ($CB_1$) receptors; for the regulation of triglyceride levels in the blood plasma; for the prophylaxis and/or treatment of disorders of the central nervous system; for the prophylaxis and/or treatment of disorders of the cardiovascular system; for the prophylaxis and/or treatment of disorders of the immune system; for the prophylaxis and/or treatment of disorders of the endocrinous system; for the prophylaxis and/or treatment of disorders of the respiratory system; for the prophylaxis and/or treatment of disorders of the gastrointestinal tract and/or for the prophylaxis and/or treatment of reproductive disorders.

The use for the preparation of a medicament for the prophylaxis and/or treatment of one or more of the following disorders:

food intake disorders, preferably selected from the group consisting of bulimia; anorexia; cachexia; psychosis; alcohol abuse and/or addiction; nicotine abuse and/or addiction; drug abuse and/or addiction; medicament abuse and/or addiction; schizophrenia; anxiety; depression; epilepsy; neurodegenerative disorders, preferably Morbus Parkinson; Morbus Huntington; Morbus Alzheimer and/or Multiple Sclerosis; cerebellar disorders; spinocerebellar disorders; cognitive disorders; cranial trauma; panic attacks; peripheric neuropathy; glaucoma; migraine; Raynaud's disease; tremblement disorders; compulsive disorders; senile dementia; thymic disorders; tardive dyskinesia; bipolar disorders; bone disorders including osteoporosis or Paget's disease of bone; cancer, preferably for the prophylaxis and/or treatment of one or more types of cancer selected from the group consisting of brain cancer; bone cancer; lip cancer; mouth cancer; esophageal cancer; stomach cancer; liver cancer; bladder cancer; pancreas cancer; ovary cancer; cervical cancer; lung cancer; breast cancer; skin camcer; colon cancer; bowl cancer and prostate cancer, more preferably for the prophylaxis and/or treatment of one or more types of cancer selected from the group consisting of colcon cancer; bowel cancer and prostate cancer, medicament-induced movement disorders; dystonia; endotoxemic shock; stroke; hemorragic shock; hypotension; insomnia; immunologic disorders; sclerotic plaques; vomiting; diarrhea; asthma; memory disorders; pruritus; pain; or for potentiation of the analgesic effect of narcotic and non-narcotic analgesics; or for influencing intestinal transit is particularly preferred.

A further aspect of the present invention relates to pharmaceutical formulations in different pharmaceutical forms comprising an inventive active substance combination according to the present invention for which (B) is not a statin, and optionally at least one further active substance and/or optionally at least one auxiliary substance.
Preferably the inventive pharmaceutical formulation is suitable for oral or parenteral administration, more preferably for oral, intravenous, intraperitoneal, intramuscular, subcutaneous, intrathekal, rectal, transdermal, transmucosal or nasal administration.
Inventive pharmaceutical formulation for oral administration are preferably selected from the group consisting of tablets, drageés, capsules, powders, drops, gels, juices, sirups, solutions and suspensions.
The pharmaceutical formulation of the present invention for oral administration may also be in the form of multiparticulates, preferably microparticles, microtablets, pellets or granules, optionally compressed into a tablet, filled into a capsule or suspended in a suitable liquid. Suitable liquids are known to those skilled in the art.
The respective pharmaceutical formulations may - depending on their route of administration - also contain one or more auxiliary substances known to those skilled in the art. The pharmaceutical formulations according to the present invention may be produced according to standard procedures known to those skilled in the art, e.g. from the tables of contents from "Pharmaceutics: the Science of Dosage Forms", Second Edition, Aulton, M.E. (Ed.) Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective descriptions are incorporated by reference and are part of the present disclosure.
In one embodiment of the present invention the pharmaceutical formulation comprises one or both of the components (A) and (B) at least partially in a sustained-release form. Preferably the inventive pharmaceutical formulation comprises component (B) at least partially in a sustained-release form.
By incorporating one or both of these components at least partially or completely in a sustained-release form it is possible to extend the duration of their effect, allowing for the beneficial effects of such a sustained release form, e.g. the maintenance of even concentrations in the blood.
Suitable sustained-release forms as well as materials and methods for their preparation are known to those skilled in the art, e.g. from the tables of contents from "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000);"Controlled Drug Delivery", Vol. I, Basic Concepts, Bruck, S.D. (Ed.), CRC Press Inc., Boca Raton (1983) and from Takada, K. and Yoshikawa, H., "Oral Drug delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encylopedia of Controlled Drug Delivery, Mathiowitz, E. (Ed.), John Wiley & Sons, Inc., New York (1999), Vol. 2, 698-728. The respective descriptions are incorporated by reference and are part of the disclosure.
If the pharmaceutical formulation according to the present invention comprises at least one of the components (A) and (B) at least partially in a sustained-release form, said sustained release may preferably be achieved by the application of at least one coating or provision of a matrix comprising at least one sustained-release material.
The sustained-release material is preferably based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.
The water-insoluble polymers used to produce a sustained-release material are preferably based on an acrylic resin, which is preferably selected from the group of poly(meth)acrylates, particularly preferably poly($C_{1-4}$)alkyl (meth) acrylates, poly($C_{1-4}$)dialkylamino($C_{1-4}$)alkyl (meth)acrylates and/or copolymers or mixtures thereof, and very particularly preferably copolymers of ethyl acrylate and methyl methacrylate with a monomer molar ratio of 2:1 (Eudragit NE30D®), copolymers of ethyl acrylate, methyl methacrylate and trimethylammonium ethyl methacrylate-chloride with a monomer molar ratio of 1:2:0.1 (Eudragit RS®), copolymers of ethyl acrylate, methyl methacrylate and trimethylammonium ethyl methacrylate-chloride with a monomer molar ratio of 1:2:0.2 (Eudragit RL®), or a mixture of at least two of the above-mentioned copolymers. These coating materials are commercially available as 30 wt.% aqueous latex dispersions, i.e. as Eudragit RS30D®, Eudragit NE30D® or Eudragit RL30D®, and may also be used as such for coating purposes.
In another embodiment, the sustained-release material is based on water-insoluble cellulose derivatives, preferably alkyl celluloses, particularly preferably ethyl cellulose, or cellulose esters, e.g. cellulose acetate. Aqueous ethyl cellulose dispersions are commercially available, for example, under the trademarks Aquacoat® or Surelease®.
As natural, semisynthetic or synthetic waxes, fats or fatty alcohols, the sustained-release material may be based on carnauba wax, beeswax, glycerol monostearate, glycerol monobehenate, glycerol ditripalmitostearate, microcrystalline wax, cetyl alcohol, cetylstearyl alcohol or a mixture of at least two of these components.

The afore mentioned polymers of the sustained-release material may also comprise a conventional, physiologically acceptable plasticizer in amounts known to those skilled in the art.

Examples of suitable plasticizers are lipophilic diesters of a $C_6$-$C_{40}$ aliphatic or aromatic dicarboxylic acid and a $C_1$-$C_8$ aliphatic alcohol, e.g. dibutyl phthalate, diethyl phthalate, dibutyl sebacate or diethyl sebacate, hydrophilic or lipophilic citric acid esters, e.g. triethyl citrate, tributyl citrate, acetyltributyl citrate or acetyltriethyl citrate, polyethylene glycols, propylene glycol, glycerol esters, e.g. triacetin, Myvacet® (acetylated mono- and diglycerides, $C_{23}H_{44}O_5$ to $C_{25}H_{47}O_7$), medium-chain triglycerides (Miglyol®), oleic acid or mixtures of at least two of said plasticizers.
Aqueous dispersions of Eudragit RS® and optionally Eudragit RL® preferably contain triethyl citrate. The sustained-release material may comprise one or more plasticisers in amounts of, for example, 5 to 50 wt.% based on the amount of polymer(s) used.

The sustained-release material may also contain other conventional auxiliary substances known to those skilled in the art, e.g. lubricants, coloured pigments or surfactants.

The pharmaceutical formulation of the present invention may also comprise at least one of the components (A) and (B) covered by an enteric coating form which dissolves as a function of pH. Because of this coating, part or all of the pharmaceutical formulation can pass through the stomach undissolved and the components (A) and/or (B) are only released in the intestinal tract. The enteric coating preferably dissolves at a pH of between 5 and 7.5.

The enteric coating may be based on any enteric material known to those skilled in the art, e.g. on methacrylic acid/methyl methacrylate copolymers with a monomer molar ratio of 1:1 (Eudragit L®), methacrylic acid/methyl methacrylate copolymers with a monomer molar ratio of 1:2 (Eudragit S®), methacrylic acid/ethyl acrylate copolymers with a monomer molar ratio of 1:1 (Eudragit L30D-55®), methacrylic acid/methyl acrylate/methyl methacrylate copolymers with a monomer molar ratio of 7:3:1 (Eudragit FS®), shellac, hydroxypropyl methyl cellulose acetate-succinates, cellulose acetate-phthalates or a mixture of at least two of these components, which can optionally also be used in combination with the above-mentioned water-insoluble poly(meth)acrylates, preferably in combination with Eudragit NE30D® and/or Eudragit RL® and/or Eudragit RS®.

The coatings of the pharmaceutical formulations of the present invention may be applied by the conventional processes known to those skilled in the art, e.g. from Johnson, J.L., "Pharmaceutical tablet coating", Coatings Technology Handbook (Second Edition), Satas, D. and Tracton, A.A. (Eds), Marcel Dekker, Inc. New York, (2001), 863-866; Carstensen, T., "Coating Tablets in Advanced Pharmaceutical Solids", Swarbrick, J. (Ed.), Marcel Dekker, Inc. New York (2001), 455-468; Leopold, C.S., "Coated dosage forms for colon-specific drug delivery", Pharmaceutical Science & Technology Today, 2(5), 197-204 (1999), Rhodes, C.T. and Porter, S.C., Coatings, in Encyclopedia of Controlled Drug Delivery. Mathiowitz, E. (Ed.), John Wiley & Sons, Inc., New York (1999), Vol. 1, 299-311. The respective descriptions are incorporated by reference and are part of the present disclosure.

In another embodiment, the pharmaceutical formulation of the present invention contains one or both of components (A) and (B) not only in sustained-release form, but also in non-retarded form. By combination with the immediately released form, a high initial dose can be achieved for the rapid onset of the beneficial effect. The slow release from the sustained release form then prevents the beneficial effect from diminishing. Such a pharmaceutical formulation is particularly useful for the treatment of acute health problems.

This may be achieved, for example, by a pharmaceutical formulation having at least one immediate-release coating comprising at least one of the components (A) and (B) to provide for rapid onset of the beneficial effect after administration to the patient.

**Pharmacological Methods**

**I. In-vitro determination of affinity to CB1 -Receptors.**

**METHODS**

Binding affinity to CB1 receptor was evaluated according to the method of Govaerts et al (2004) with the following modifications. Cerebella from male Wistar rats (250-300g) were carefully dissected on ice and homogenates were prepared in cold 50 mM Tris-HCl, 5 mM $MgCl_2$, 1 mM EDTA, 0.25 M sucrose, pH 7.4 with a Potter-Heljheim homogenizer and the suspension was centrifuged at 1,000 x g for 5 minutes. The supernatants were collected and centrifuged at 50,000 x g for 15 min. The resulting pellets were resuspended in Tris-HCl buffer without sucrose, homogenized and incubated for 15 min at 37°C in an orbital shaker bath and centrifuged again at 50,000 x g for 15 min. Pellets were weighed, resuspended in Tris-HCl buffer without sucrose, homogenized with an Ultraturrax at 13,500 rpm for 3 x 5 seconds and aliquoted in 0.9 ml volumes in Eppendorf tubes. Aliquots were centrifuged at 20,800 x g for 5 minutes, supernatants discarded and pellets were frozen at -80°C until use. Total protein concentration was determined using a Bio-Rad commercial assay base on the Lowry method. Competitive binding experiments were performed in presence of 1 nM [$^3$H]-CP 55,940 in siliconized glass tubes containing 100 μg protein/tube resuspended in 1ml final volume of 50

mM Tris-HCl, 5 mM $MgCl_2$ , 1 mM EDTA, 0.5% (w/v) bovine serum albumin, pH 7.4. Competitors were present at various concentrations and the non-specific binding was determined in the presence of 10 μM HU-210. After 1 hour incubation at 30°C, the suspension was rapidly filtered through 0.5% PEI pre-treated GF/B fiber filters on a 96-well harvester and washed 3 times with 3ml ice-cold binding buffer without bovine serum albumin. Radioactivity on filters was measured with Wallac Winspectral 1414 counter by liquid scintillation in 6 ml Ecoscint H (National Diagnostics, U.K.). Assays were made in triplicate.

Binding data were analyzed by non-linear regression with the software GraphPad Prism Version 3.03.

## RESULTS

Affinities of pyrazolines compounds according to the present invention, for the rat CB1 receptor subtype were determined in vitro, Compound of examples 1 and 8 were found to have moderate affinity for these receptors with a $IC_{50}$ values of 26 nM and 22 nM, respectively. On the other hand, with an $IC_{50}$ value of 813 nM, Compound of example 9 has less affinity for rat CB1 receptors.

## II. Effect of combination according to the invention on plasma lipid profiles and on other metabolic disturbances associated with the aetiology of dyslipidaemia.

## METHODS

### Experiment in female, dietary-induced (DIO), female Wistar rats

### Animals

The experiment was performed on female Wistar rats (originally in the weight range 250-300g). Animals were maintained on a reverse phase light-dark cycle (lights off for 8 h from 09.30-17.30 h) during which time the room was illuminated by red light. Animals had free access to powdered high fat diet (VRF1 plus 20% lard), ground chocolate, ground peanuts and tap water at all times. The three different diets were contained in separate glass feeding jars with aluminium lids with a 3-4 cm hole cut in it to allow access to the food. Animals were housed in pairs for twelve weeks for the induction of obesity. Animals were then housed singly in polypropylene cages with wire grid floors to enable the food intake of each rat to be recorded. Polypropylene trays with cage pads were placed beneath each cage to detect any food spillage. Animals were accustomed to these new conditions (individual housing and the wire-grid floor cages) for at least 14 days before the start of the baseline readings.

### Experimental procedures for the feeding study

Approximately a week before the start of the study, animals were weighed (to the nearest 0.1 g using an electronic top-pan balance) and allocated into 2 weight-matched treatment groups, each containing 10 animals. Following a 7 day baseline run-in period, during which the animals were dosed by the appropriate route (eg orally) once a day with vehicle (1 % methylcellulose; 1 ml/kg) or the test drug (30 mg/kg po) once daily for 28 days.

On the first day of drug administration food intake was also measured at 2 and 6 h as well as at 24h. Thereafter food intake and body weight were measured once daily. Variations in body weight and energy levels of the different types of food were accounted for by expressing the food intake results in terms of kJ/kg rat weight. Results for food intake were statistically analysed using Student's 1-tailed t-test with the significance set at $p < 0.05$. Body weights were measured once daily.

Statistical comparisons between the body weights of the different treatment groups have been made by analysis of covariance followed by multiple t tests (two-tailed) to compare each treatment group with the vehicle-treated controls. For the body weight data (body weights each day), the weights of the animals on Day 1 (ie immediately-before the first drug treatment) were used as the covariate. Thus, means were adjusted for differences in body weight between the groups on Day 1 and standard errors of the mean (SEM) were calculated from the residuals of the statistical model. All mean body weight values on Day 1 are identical (and SEM are therefore zero) because the analysis of covariance adjusts them all to equal the 'grand mean' of the values of this day.

### Plasma analyses

Terminal blood samples were taken via cardiac puncture from the animals immediately after death into Sarstedt 4.5 ml lithium-heparin tubes (Cat No. 32.331). Tubes were then centrifuged at 1,500 g for 5 minutes at 4°C and the plasma removed and immediately frozen on dry ice (as 3 aliquots per sample in sealed Eppendorf-tubes). Plasma

samples were stored at -75°C until required for analysis. All determinations were performed on plasma samples that had been thawed only once.

**Glycerol and triacylglycerol assay**

Plasma triacylglycerol was determined by modification of a discrete assay kit (Sigma Triglyceride determination kit, Cat No. TR0100) to a 96-well format. The kit actually determines liberated glycerol following enzymic hydrolysis of triacylglycerol to free fatty acids and glycerol (1 mole glycerol per mole of triacylglycerol). For this reason, correction for the glycerol concentration originally present in the plasma is required. Plasma samples were, therefore, assayed in the absence (free plasma glycerol) or presence (total triacylglycerol) of lipoprotein lipase. Subtraction of free glycerol from total triacylglycerol concentration then derives the true triacylglycerol concentration.

A 2.5 mg/ml triolein equivalent glycerol standard was obtained from Sigma. The supplied Sigma mM conversion factor for this batch was 1.13 (ie 2.83 mM glycerol). A 10 mg/ml glycerol stock solution was prepared in saline and then diluted in saline to create a series of standard concentrations from 0.218 to 4.34 mM. Standards and plasma samples (undiluted) were pipetted into the 96-well plates as 10 μl additions in duplicate. Assay plates and reagents were preheated at 37°C and the reaction started by adding 200 μl of either the glycerol or total triacylglycerol reagent (reconstituted as per manufacturer's protocol) followed by incubation at 37°C for 5 minutes after which optical density at 540 nm was determined using a Molecular Devices VERSAmax tunable microplate reader. Optical density readings were then transferred to GraphPad Prism and unknown values extrapolated from a linear regression fit to the standard curve. The assay was linear up to the highest glycerol standard employed (4.34 mM). Subtraction of the glycerol concentration in the plasma samples from the total triacylglycerol concentration derived the true triacylglycerol concentration.

**Leptin assay**

Plasma leptin concentration was determined using Assay Designs rat leptin enzyme immunometric assay kits (Cat No. 900-015) according to the manufacturer's regular protocol. Unknown samples were diluted 100 times using leptin assay buffer prior to assay in duplicate. Optical density at 450 nm was determined using a Molecular Devices VERSAmax tunable microplate reader and readings transferred to GraphPad Prism. A second order polynomial curve was fitted to the calibration data and unknown values extrapolated from this curve fit.

**Insulin assay**

Plasma insulin concentration was determined using Mercodia ultrasensitive rat insulin ELISA kits according to the manufacturers 50 μl sample protocol. Unknown samples were diluted 20 times using the zero standard prior to assay in duplicate. Optical density at 450 nm was determined using a Molecular Devices VERSAmax tunable microplate reader and readings transferred to GraphPad Prism. A cubic spline curve was fitted to the calibration data and unknown values extrapolated from this curve fit.

**Determination of carcass fat content**

Total ether-extractable fat in the freeze-dried samples was estimated by the Soxhlet principle using a Foss Soxtec HT2 system. The protocol employed was based on the manufacturer's recommendations for meat samples, although freeze-dried samples obviated the necessity for either a fat hydrolysis step or mixing the sample with sand to ensure efficient extraction.

Samples of approximately 1 g were accurately weighed into 26 mm x 60 mm cellulose extraction thimbles which were then plugged with approximately 0.8 g cotton wool. Sample fat was extracted into pre-weighed (including a few boiling chips) standard metal extraction cups using 70 ml of petroleum ether at 40-60°C, a circulator temperature of 115°C and a cooling flow rate of 2 l/min. A boiling stage of 25 min was employed, followed by a 40 min wash stage. A 10 min drying stage sufficed to remove ether from the extraction cups.

Percentage fat in the freeze-dried sample was calculated as follows:

$$\% \text{ fat} = [100 \times (\text{final cup weight} - \text{initial cup weight})]/\text{sample weight}$$

Percentage fat in the original sample was calculated using the % water as follows:

$$\% \text{ fat in original sample} = \% \text{ fat} \times [(100 - \% \text{ water})/100]$$

**Determination of carcass protein content**

Total protein in the freeze-dried samples was estimated using the Kjeldahl principle. A micro-Kjeldahl procedure was performed using a Foss 2012 digestion block and a Foss 2200 distilling unit. The protocol used was based on the manufacturer's recommendations for meat samples.

Approximately 0.3 g of freeze-dried sample was accurately weighed into 100 ml digestion tubes (Foss UK Ltd) in racks of 12 tubes. Each rack included at least one blank tube to determine a reagent blank. To each tube was added two Kjeltabs CQ and one Antifoam S tablet followed by 10 ml of 98% sulphuric acid. Samples were digested at 420°C for 1 h to convert protein into ammonium sulphate. Once tubes had cooled, free ammonia was liberated by the addition of an excess of 40% NaOH and steam distilled into 30 ml of 4% boric acid indicator solution. Ammonia was then determined by titration against volumetric grade 0.1 M HCl using a Brand 25 ml digital burette. Nitrogen and protein content of the samples were calculated as follows:

$$\% \text{ nitrogen} = [(\text{sample titration} - \text{blank titration}) \times 0.1401]/\text{sample weight (g)}$$

$$\% \text{ protein} = \% \text{ nitrogen} \times 6.25$$

**Determination of carcass ash content**

Total ash in the freeze-dried samples was estimated by determining the non-combustible ash remaining after firing samples at high temperatures using a Carbolite OAF 11/1 muffle furnace.

Squat form silica crucibles were prefired (600°C) for at least 2 h to stabilise weights. Crucibles were allowed to cool (<300°C) and transferred to silica gel drying cabinets. Crucibles were then weighed, approximately 1 g of sample added and the crucible reweighed. A number of empty crucibles were also included. Crucibles were fired at 600°C for 4 h to convert samples to ash and then allowed to cool (<300°C) prior to transfer to silica gel drying cabinets. Final weights were determined when crucibles had fully cooled.

Percentage ash was determined by expressing the residual sample weight after firing as a percentage of the original sample weight. The average variation in blank crucible weight was -0.02 ± 0.04 mg (mean ± SEM, n=14) and individual weights varied by 0.5 mg or less.

**Experiment in male, dietary-induced (DIO) Sprague-Dawley rats**

The experiment was performed on selectively bred, male, Sprague-Dawley rats that were assessed at 3-5 weeks of age as having a predisposition to become obese. These animals were maintained on a normal light cycle (Lights on between 06.00h and 18.00h) under controlled temperature conditions. These rats were then given ad lib access to a commercial, energy-dense, high-fat diet. The animals for the study were 24 weeks old at the start of the experiment.

Groups of rats were injected intraperitoneally with active substance combination of the present invention (10 mg/kg in 0.1% Tween in saline; n = 15) or vehicle (0.1% Tween in saline; n = 10). Animals were injected daily between 09.00h and 11.00h and food intake and body-weights were measured daily at the time of injection from Day 0.

**Oral glucose tolerance test**

On Day 21, groups of compound- and vehicle-treated mildly fasted rats were administered 2 g/kg glucose (as a 2g/ml solution) via a gastric tube placed into the stomach. Blood samples for plasma insulin determinations were taken at -30 min, 0, 15, 30, 60 and 120 min.

**Blood sampling**

Blood samples were taken from the tail veins of mildly fasted rats (restricted to 50% of their normal food intake in the 20h period before sampling) for the determination of plasma concentrations of triglycerides FFAs and insulin on Day 1 and Day 21.

**Determination of plasma lipid and insulin concentrations**

Plasma concentrations of triacylglycerol were determined using a standard automated enzymatic assay and FFAs were quantified using an acyl-CoA oxidase based colourimetric assay. Plasma insulin was determined by an ultra-sensitive ELISA method.

**Fat depots analysis**

On the day of termination of the experiment, inguinal, epidiymal, mesenterial and retroperitoneal fat depots were removed and weighed.

**Statistical analyses**

Calculations and statistical analyses were performed using GraphPad and Statview software packages. One-way ANOVA followed by Fischer's post hoc test was used to analyse the experimental data. $P < 0.05$ was set as the acceptance level for significant difference between groups.

**RESULTS:**

Active substance combination according to the present invention induces an improved lipid profile in female, DIO Witsar rats. It also induces a significant reduces of the food intake.
Aslo was observed a marked reduction in overall adiposity, as well as in plasma insulin levels.

**III. Effects of combination according to the present invention on dyslipidaemia food intake, body weight, adiposity, and insulin resistance in rats that have become obese through the excessive consumption of palatable foods sources**

**METHODS**

**Animals**

The experiment was performed on female Wistar rats (originally in the weight range 250-300 g). The rats were housed in pairs in polypropylene cages with solid floors and sawdust bedding at a temperature of 21±4°C and 55±20% humidity. Animals were maintained on a reverse phase light-dark cycle (lights off for 8 h from 09.30-17.30 h) during which time the room was illuminated by red light. Animals had free access to powdered high fat diet (VRF1 plus 20% lard), ground chocolate, ground peanuts and tap water at all times. The three different diets were contained in separate glass feeding jars with aluminum lids with a 3-4 cm hole cut in it to allow access to the food. Animals were housed in pairs for twelve weeks for the induction of obesity. Animals were then housed singly in polypropylene cages with wire grid floors to enable the food intake of each rat to be recorded. Polypropylene trays with cage pads were placed beneath each cage to detect any food spillage. Animals were accustomed to these new conditions (individual housing and the wire-grid floor cages) for at least 14 days before the start of the baseline readings.

**Experimental procedures for the feeding study**

Approximately a week before the start of the study, animals were weighed (to the nearest 0.1 g using an electronic top-pan balance) and allocated into 3 weight-matched treatment groups, each containing 9-10 animals. Following a 7 day baseline run-in period, during which the animals were dosed by the appropriate route (eg orally) once a day with vehicle (0.5% hydroxy-propyl-methylcellulose [HPMC]; 1 ml/kg) or the test drug, ie E-9129 (10 and 30 mg/kg po) and E-9130 (10 and 30 mg/kg po) once daily for 28 days.
Food intake and body weight were measured once daily. Variations in body weight and energy levels of the different types of food were accounted for by expressing the food intake results in terms of kJ/kg rat weight.
Statistical comparisons between the body weights and daily food intakes of the different treatment groups have

been made by analysis of covariance followed by Dunnett's test to compare each treatment group with the vehicle-treated controls. For the body weight data (body weights each day), the weights of the animals on Day 1 (ie immediately before the first drug treatment) were used as the covariate. Thus, means were adjusted for differences in body weight between the groups on Day 1 and standard errors of the mean (SEM) were calculated from the residuals of the statistical model. All mean body weight values on Day 1 are identical (and SEM are therefore zero) because the analysis of covariance adjusts them all to equal the 'grand mean' of the values of this day.

**Plasma analyses**

Terminal blood samples were taken via cardiac puncture from the animals immediately after death into Sarstedt 4.5 ml lithium-heparin tubes (Cat No. 32.331). Tubes were then centrifuged at 1,500 g for 5 minutes at 4°C and the plasma removed and immediately frozen on dry ice (as 3 aliquots per sample in sealed Eppendorf tubes). Plasma samples were stored at -75°C until required for analysis. All determinations were performed on plasma samples that had been thawed only once.

**NEFA assay**

Non-esterified (or free) fatty acids (NEFA) were determined by modification of a commercial clinical analyser assay kit (Wako NEFA C ACS-ACOD method) to a 96-well format. The supplied oleic acid standard (1 mM) was diluted in deionised water to create a series of standard concentrations from 50 μM to 1000 μM. Plasma samples were assayed after a 2 times dilution in deionised water. Standards and diluted plasma samples were pipetted into the plates (Alpha Laboratories, flat bottomed FX9200) as 10 μl additions in duplicate. Assay plates and reagents A and B were preheated at 37°C and the reaction started by adding 75 μl of reagent A followed by incubation at 37°C. After exactly 10 minutes, 150 μl of reagent B was added and the plate incubated for a further 10 minutes after which optical density at 540 nm was determined using a Molecular Devices VERSAmax tunable microplate reader. Optical density readings were then transferred to GraphPad Prism and values for plasma samples extrapolated from a linear regression fit to the standard curve. The assay was linear up to the highest standard employed (1000 μM).

**Glycerol and triacylglycerol assay**

Plasma triacylglycerol was determined by modification of a discrete assay kit (Sigma Triglyceride determination kit, Cat No. TR0100) to a 96-well format. The kit actually determines liberated glycerol following enzymic hydrolysis of triacylglycerol to free fatty acids and glycerol (1 mole glycerol per mole of triacylglycerol). For this reason, correction for the glycerol concentration originally present in the plasma is required. Plasma samples were, therefore, assayed in the absence (free plasma glycerol) or presence (total triacylglycerol) of lipoprotein lipase. Subtraction of free glycerol from total triacylglycerol concentration then derives the true triacylglycerol concentration.

A 2.5 mg/ml triolein equivalent glycerol standard was obtained from Sigma. The supplied Sigma mM conversion factor for this batch was 1.13 (ie 2.83 mM glycerol). A 10 mg/ml glycerol stock solution was prepared in saline and then diluted in saline to create a series of standard concentrations from 0.218 to 4.34 mM. Standards and plasma samples (undiluted) were pipetted into the 96-well plates as 10 μl additions in duplicate. Assay plates and reagents were preheated at 37°C and the reaction started by adding 200 μl of either the glycerol or total triacylglycerol reagent (reconstituted as per manufacturer's protocol) followed by incubation at 37°C for 5 minutes after which optical density at 540 nm was determined using a Molecular Devices VERSAmax tunable microplate reader. Optical density readings were then transferred to GraphPad Prism and unknown values extrapolated from a linear regression fit to the standard curve. The assay was linear up to the highest glycerol standard employed (4.34 mM). Subtraction of the glycerol concentration in the plasma samples from the total triacylglycerol concentration derived the true triacylglycerol concentration.

**Leptin assay**

Plasma leptin concentration was determined using Assay Designs rat leptin enzyme immunometric assay kits (Cat No. 900-015) according to the manufacturer's regular protocol. Unknown samples were diluted 100 times using leptin assay buffer prior to assay in duplicate. Optical density at 450 nm was determined using a Molecular Devices VERSAmax tunable microplate reader and readings transferred to GraphPad Prism. A second order polynomial curve was fitted to the calibration data and unknown values extrapolated from this curve fit.

**Insulin assay**

Plasma insulin concentration was determined using Mercodia ultrasensitive rat insulin ELISA kits according to

the manufacturers 50 μl sample protocol. Unknown samples were diluted 20 times using the zero standard prior to assay in duplicate. Optical density at 450 nm was determined using a Molecular Devices VERSAmax tunable microplate reader and readings transferred to GraphPad Prism. A cubic spline curve was fitted to the calibration data and unknown values extrapolated from this curve fit.

**Determination of carcass fat content**

Total ether-extractable fat in the freeze-dried samples was estimated by the Soxhlet principle using a Foss Soxtec HT2 system. The protocol employed was based on the manufacturer's recommendations for meat samples, although freeze-dried samples obviated the necessity for either a fat hydrolysis step or mixing the sample with sand to ensure efficient extraction.

Samples of approximately 1 g were accurately weighed into 26 mm x 60 mm cellulose extraction thimbles which were then plugged with approximately 0.8 g cotton wool. Sample fat was extracted into pre-weighed (including a few boiling chips) standard metal extraction cups using 70 ml of petroleum ether at 40-60°C, a circulator temperature of 115°C and a cooling flow rate of 2 l/min. A boiling stage of 25 min was employed, followed by a 40 min wash stage. A 10 min drying stage sufficed to remove ether from the extraction cups.

Percentage fat in the freeze-dried sample was calculated as follows:

$$\% \text{ fat} = [100 \times (\text{final cup weight} - \text{initial cup weight})]/\text{sample weight}$$

Percentage fat in the original sample was calculated using the % water as follows:

$$\% \text{ fat in original sample} = \% \text{ fat} \times [(100 - \% \text{ water})/100]$$

**Determination of carcass protein content**

Total protein in the freeze-dried samples was estimated using the Kjeldahl principle. A micro-Kjeldahl procedure was performed using a Foss 2012 digestion block and a Foss 2200 distilling unit. The protocol used was based on the manufacturer's recommendations for meat samples.

Approximately 0.3 g of freeze-dried sample was accurately weighed into 100 ml digestion tubes (Foss UK Ltd) in racks of 12 tubes. Each rack included at least one blank tube to determine a reagent blank. To each tube was added two Kjeltabs CQ and one Antifoam S tablet followed by 10 ml of 98% sulphuric acid. Samples were digested at 420°C for 1 h to convert protein into ammonium sulphate. Once tubes had cooled, free ammonia was liberated by the addition of an excess of 40% NaOH and steam distilled into 30 ml of 4% boric acid indicator solution. Ammonia was then determined by titration against volumetric grade 0.1 M HCl using a Brand 25 ml digital burette. Nitrogen and protein content of the samples were calculated as follows:

$$\% \text{ nitrogen} = [(\text{sample titration} - \text{blank titration}) \times 0.1401]/\text{sample weight (g)}$$

$$\% \text{ protein} = \% \text{ nitrogen} \times 6.25$$

### Determination of carcass ash content

Total ash in the freeze-dried samples was estimated by determining the non-combustible ash remaining after firing samples at high temperatures using a Carbolite OAF 11/1 muffle furnace.
Squat form silica crucibles were prefired (600°C) for at least 2 h to stabilise weights. Crucibles were allowed to cool (<300°C) and transferred to silica gel drying cabinets. Crucibles were then weighed, approximately 1 g of sample added and the crucible reweighed. A number of empty crucibles were also included. Crucibles were fired at 600°C for 4 h to convert samples to ash and then allowed to cool (<300°C) prior to transfer to silica gel drying cabinets. Final weights were determined when crucibles had fully cooled.
Percentage ash was determined by expressing the residual sample weight after firing as a percentage of the original sample weight. The average variation in blank crucible weight was -0.02 ± 0.04 mg (mean ± SEM, n=14) and individual weights varied by 0.5 mg or less.

### RESULTS

Active combination according to the present invention demonstrates a significant action in decreasing food intake, body weight, adiposity, and insulin resistance in rats that have become obese through the excessive consumption of palatable foods sources. This is particularly true when using compounds of examples 8 and 9.

## IV. Effect of repeated administration of a combination according to the invention on drug-induced weight-gain.

### METHODS

### <u>Experiment in female Sprague-Dawley rats</u>

### Animals

The experiment was performed on 40 female Sprague-Dawley rats (originally in the weight range 250-300 g). Animals were individually housed in polypropylene cages with metal grid floors. The animal room was maintained at a temperature of 21±4°C and 55±20% humidity and on a reverse phase light-dark cycle (lights off for 8 h from 09.30-17.30 h) during which time the room was illuminated by red light. Animals had free access to powdered high fat diet (VRF1 plus 20% lard). The diet was contained in a glass feeding jar with an aluminium lid with a 3-4 cm hole cut in it to allow access to the food. Animals were housed singly in polypropylene cages with wire grid floors to enable the food intake of each rat to be recorded. Polypropylene trays with cage pads were placed beneath each cage to detect any food spillage. Animals were accustomed to these new conditions (individual housing and the wire-grid floor cages) for at least 14 days before the start of the baseline readings.

### Experimental procedures for the feeding study

Approximately a week before the start of the study, animals were weighed (to the nearest 0.1 g using an electronic top-pan balance) and allocated into 4 weight-matched treatment groups, each containing 10 animals, ie vehicle (0.5% HPMC (hydroxyl-propyl-methyl-cellulose) 3ml/kg po), olanzapine (3 mg/kg po in 0.5% HPMC), active substance combination (30 mg/kg po in 0.5% HPMC), and olanzapine (3 mg/kg po in 0.5% HPMC), + active substance combination (30 mg/kg po in 0.5% HPMC). Following a 7 day baseline run-in period, during which the animals were dosed with orally with vehicle to acclimatise them to dosing/handling, the treatments above were then given once daily for 14 days.
Food intake and body weight were measured once daily. Variations in body weight were accounted for by expressing the food intake results in terms of g/kg rat weight.
Statistical comparisons between the food intakes of the different treatment groups were made by analysis of covariance followed by multiple t tests (two-tailed) to compare each treatment group with the vehicle-treated controls. Food intake of the animals at baseline (ie average of Day -6 to 0) was used as the covariate. Standard errors of the mean (SEM) were calculated from the residuals of the statistical model.
Statistical comparisons between the body weights of the different treatment groups were made by analysis of covariance followed by multiple t tests (two-tailed) to compare each treatment group with the vehicle-treated controls. For the body weight data (body weights each day), the weights of the animals on Day 1 (ie immediately before the first drug treatment) were used as the covariate. Thus, means were adjusted for differences in body weight between the groups on Day 1 and standard errors of the mean (SEM) were calculated from the residuals of the statistical model. All mean body weight values on Day 1 are identical (and SEM are therefore zero) because the analysis of covariance adjusts them all to equal the 'grand mean' of the values of this day.

**RESULTS**

The results clearly demonstrate that bodyweight and food intake were significantly decreased after weight gain and hyperphagia induced by olanzapine.

**Examples:**

**Example 1:**

**N-piperidinyl-5-(4-chlorophenyl)-1-(2,4-dichlorophenyl)-4,5-dihydro-1H-pyrazole-3-carboxamide**

**a) 4-(4-chlorophenyl)-2-oxo-3-butenoic acid**

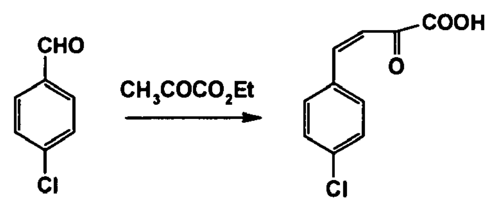


In a three neck flask p-chlorobenzaldehyde (13,3 g, 95 mmoles) and ethyl pyruvate (10 g, 86 mmoles) were dissolved in 150 ml of absolute ethanol.The solution was ice-cooled to 0°C and an aqueous solution of NaOH (3.8 g in 45 mL water) was added dropwise keeping the temperature below or equal to 10°C, whereby a yellow-orange colored precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).

IR (KBr, $cm^{-1}$) : 3500-2500, 1719,3, 1686,5, 1603,4, 1587,8, 1081,9.

$^1H$ NMR($CDCl_3$, $\delta$) : 7,4 (d, J=8,4Hz, 2H), 7,5 (d, J=16,1 Hz, 1 H), 7,6 (d, J=8,4Hz, 2H), 8,1(d, J=16,1Hz, 1H).

**b) 5-(4-chlorophenyl)-1-(2,4-dichlorophenyl)-4,5-dihydro-pyrazole-3-carboxylic acid**

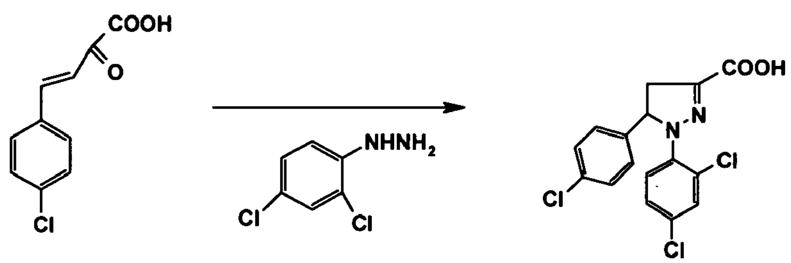

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride

fractions were washed with water, dried with sodium sulfate, filtered and evaporated to dryness to give a pale yellow solid (12.7 g, 57% of theoretical yield).

IR (KBr, $cm^{-1}$): 3200-2200, 1668,4, 1458, 1251,4, 1104,8.

$^1H$ NMR ($CDCl_3$, δ) : 3,3 (dd, 1 H), 3,7 (dd, 1 H), 5,9 (dd, 1 H), 7,09-7,25 (m, 7H).

**(c) 5-(4-chlorophenyl)-1-(2,4-dichlorophenyl)-4,5-dihydro-pyrazole-3-carboxylic acid chloride**

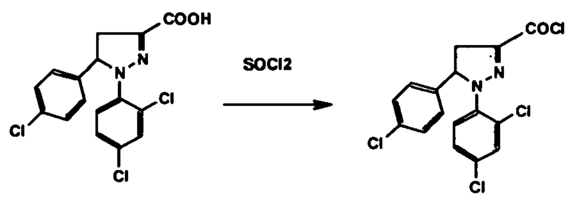


Under nitrogen atmosphere 5-(4-chlorophenyl)-1-(2,4-dichlorophenyl)-4,5-dihydro-pyrazole-3-carboxylic acid (2.5 g, 6.8 mmols) obtained according to step (b) was dissolved in 4 mL of in thionyl chloride and heated to reflux for 2.5 hours. The excess thionyl chloride is removed from the reaction mixture under reduced pressure and the resulting crude residue (2.6 g) is used without any further purification.

IR (KBr, $cm^{-1}$): 1732,3, 1700, 1533,3, 1478,1, 1212,9, 826,6.

**d) N-piperidinyl-5-(4-chlorophenyl)-1-(2,4-dichlorophenyl)-4,5-dihydropyrazole-3-carboxamide** [this compound may also be referred to as 5-(4-Chloro-phenyl)-1-(2,4-dichloro-phenyl)-4,5-dihydro-1H-pyrazole-3-carboxylic acid piperidin-1-ylamide or as 1-(2,4-dichlorophenyl)5-(4-chlorophenyl)-4,5-dihydro-N-(piperidin-1-yl)-1H-pyrazole-3-carboxamide]

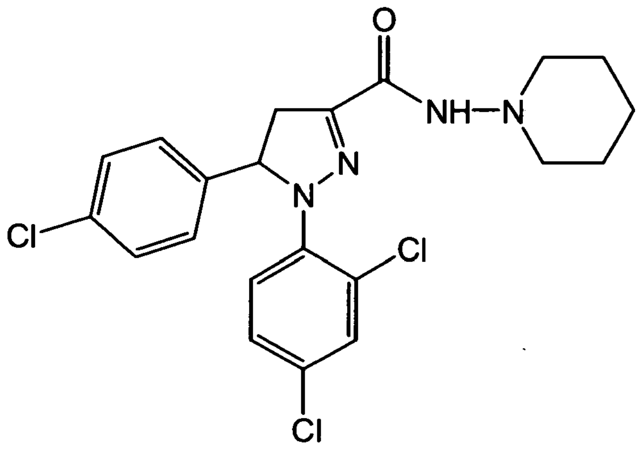

Under nitrogen atmosphere N-aminopiperidine (0.6 mL, 5.6 mmoles) and triethylamine (4 mL) were dissolved in methylene chloride (25 mL). The resulting mixture was ice-cooled down to 0°C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of

1.7 g (57% of theoretical yield) of N-piperidinyl-5-(4-chlorophenyl)-1-(2,4-dichlorophenyl)-4,5-dihydropyrazole-3-carboxamide having a melting point of 183-186°C.

IR (KBr, $cm^{-1}$) : 3222,9, 2934,9, 1647,4, 1474,7, 1268,3, 815,6.

$^1$H NMR ($CDCl_3$, δ) : 1,4 (m, 2H), 1,7 (m, 4H), 2,8 (m, 4H), 3,3 (dd, J=6,1 y 18,3Hz, 1 H), 3,7 (dd, J=12,5 and 18,3 Hz, 1 H), 5,7 (dd, J=6,1 and 12,5 Hz, 1 H), 7,0-7,2 (m, 6H), 7,4 (s, 1H).

The compounds according to the following examples 2-6 have been prepared analogously to the process described in Example 1.

**Example 2:**

**5-(4-Chloro-phenyl)-1-(2,4-dichloro-phenyl)-4,5-dihydro-1H-pyrazole-3-carboxylic acid-[1,2,4]triazol-4-yl amide**

Melting point: 134-138 °C.

IR (KBr, cm-$^1$): 3448, 1686, 1477, 1243, 1091, 821.

$^1$H NMR($CDCl_3$, δ): 3,1 (dd, J=6,2 and 17,9Hz, 1 H), 3,7 (dd, J=12,3 and 17,9Hz, 1 H), 5,9 (dd, J=6,2 and 12,3 Hz, 1 H), 7,2-7,5 (m, 7H), 8,7 (s, 2H), 12,0 (bs, 1H).

**Example 3:**

**5-(4-Chloro-phenyl)-1-(2,4-dichloro-phenyl)-4,5-dihydro-1H-pyrazole-3-carboxylic acid-(4-methyl-piperazin-1-yl)-amide hydrochloride**

Melting point: 150-155ºC.

IR (KBr, $cm^{-1}$): 3433, 1685, 1477, 1296, 1246, 1088, 1014, 825.

$^1$H NMR ($CDCl_3$, δ): 2,7 (d, J=4,2Hz, 3H), 3,0-3,4 (m, 9H), 3,6 (dd, J=11,9 and 17,9 Hz, 1 H), 5,8 (dd, J=5,5 and 11,9 Hz, 1 H), 7,1 (d, J=8,4Hz, 2H), 7,25 (2d, J= 8,4 and 8,7 Hz, 3H), 7,4 (d, J=2,2Hz, 1 H), 7,5 (d, J=8,7Hz, 1 H), 9,8 (s, 1H), 11,2 (bs).

**Example 4:**

**5-(4-Chloro-phenyl)-1-(2,4-dichloro-phenyl)-4,5-dihydro-1 H-pyrazole-3-carboxylic acid diethylamide**

This compound was obtained in form of an oil.

IR (film, $cm^{-1}$) : 2974, 1621, 1471, 1274, 1092, 820.

$^1$H NMR ($CDCl_3$, δ): 1,2 (m, 6H), 3,3-3,9 (m, 6H), 5,6 (dd, J=5,8 and 11,7 Hz, 1 H), 7-7,25 (m, 7H).

**Example 5:**

**[5-(4-Chloro-phenyl)-1-(2,4-dichloro-phenyl)-4,5-dihydro-1H-pyrazol-3-yl]-piperidin-1-yl-methanone**

Melting point: 105-110ºC.

IR (KBr, $cm^{-1}$) : 2934, 1622, 1470, 1446, 1266, 1010, 817.

$^1$H NMR ($CDCl_3$, δ): 1,7 (m, 6H), 3,4 (dd, J=5,7 and 17,9Hz, 1 H), 3,7 (m, 3H), 3,9 (m, 2H), 5,6 (dd, J=6,1 y 11,9 Hz, 1H), 7-7,25 (m, 7H).

**Example 6:**

**N-[5-(4-Chloro-phenyl)-1-(2,4-dichloro-phenyl)-4,5-dihydro-1H-pyrazole-3-carbonyl]-4-methyl-phenylsulfonamide**

This compound was obtained in form of an amorph solid.

IR (KBr, $cm^{-1}$): 1697, 1481, 1436, 1340, 1169, 1074, 853.

$^1$H NMR ($CDCl_3$, δ): 2,4 (s, 3H), 3,2 (dd, J=6,6 and 18,3Hz, 1H), 3,6 (dd, J=12,8 and 18,3Hz, 1 H), 5,8 (dd, J=6,6 and 12,8Hz, 1 H), 7 (d, J=8,2Hz, 2H), 7,2 (s, 1 H), 7,3-7,4 (m, 6H), 8 (d, J=8,1 Hz, 2H), 9 (s, 1 H).

**Example 7:**

N-oxide of N-piperidinyl-5-(4-chlorophenyl)-1-(2,4-dichlorophenyl)-4,5-dihydropyrazole-3-carboxamide

Under nitrogen gas as an inert atmosphere N-piperidinyl-5-(4-chlorophenyl)-1-(2,4-dichlorophenyl)-4,5-dihydropyrazole-3-carboxamide (0,15 g, 332 mmoles) was dissolved in 7 ml of dichloromethane. The resulting solution was ice-cooled to 0 °C and m-chloroperbenzoic acid (0,204 g, 0,83 mmoles) added in several portions. After stirring for 15 minutes a control via thin layer chromatography showed that no starting material was remaining. A saturated solution of sodium bicarbonate was then slowly added, the organic phase separated, washed with water, dried over sodium sulfate and filtered. The filtered solution was evaporated to dryness and the crude product was purified via column chromatography yielding 78 mg (50 % of theoretical yield) of the N-oxide of N-piperidinyl-5-(4-chlorophenyl)-1-(2,4-dichlorophenyl)-4,5-dihydropyrazole-3-carboxamide in form of a white solid having a melting point of 115-120 °C.

IR (KBr, $cm^{-1}$): 3202, 1678, 1654, 1474, 1309, 1107.

$^1H$-NMR ($CDCl_3$, δ): 1.6 (m, 2H), 1.8-2.0 (m, 4H), 2.55 (m, 2H), 3.3 (dd, J = 6.3 Hz and 18.2 Hz, 1 H), 3.7 (m, 3H), 5.8 (dd, J = 6.3 Hz and 12.5 Hz, 1 H), 7.0-7.3 (m, 7H), 8.5 (s, 1H.)

The following examples 8-11 where obtained by resolution of the corresponding racemic mixtures :

**Example 8** : (R)-N-piperidinyl-5-(4-chlorophenyl)-1-(2,4-dichlorophenyl)-4,5-dihydro-1H-pyrazole-3-carboxamide.

**Example 9** : (S)-N-piperidinyl-5-(4-chlorophenyl)-1-(2,4-dichlorophenyl)-4,5-dihydro-1H-pyrazole-3-carboxamide.

**Example 10** : (R)-5-(4-chlorophenyl)-1-(2,4-dichlorophenyl)-4,5-dihydro-pyrazole-3-carboxylic acid.

**Example 11** : (S)-5-(4-chlorophenyl)-1-(2,4-dichlorophenyl)-4,5-dihydro-pyrazole-3-carboxylic acid.

**Claims**

**1.** Active substance combination comprising

(A) at least one substituted pyrazoline compound of general formula I

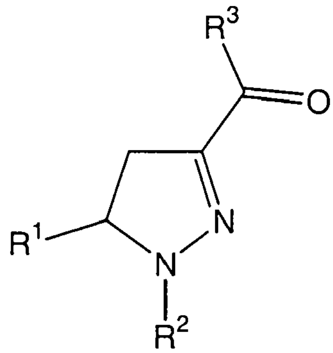

I

wherein

$R^1$ represents an optionally at least mono-substituted phenyl group;

$R^2$ represents an optionally at least mono-substituted phenyl group;

$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an - $NR^4R^5$-moiety,

$R^4$ and $R^5$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group; an $-SO_2-R^6$-moiety; or an $-NR^7R^8$-moiety, with the proviso that $R^4$ and $R^5$ do not identically represent hydrogen;

$R^6$ represents a linear or branched, saturated or unsaturated, optionally at least mono-substituted aliphatic group; a saturated or unsaturated, optionally at least mono-substituted, optionally at least one heteroatom as ring member containing cycloaliphatic group, which may be condensed with a mono- or polycyclic ring-system; or an optionally at least mono-substituted aryl or heteroaryl group, which may be condensed with a mono- or polycyclic ring system and/or bonded via a linear or branched alkylene group;

$R^7$ and $R^8$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and/or

at least one substituted pyrazoline compound of general formula 1’,

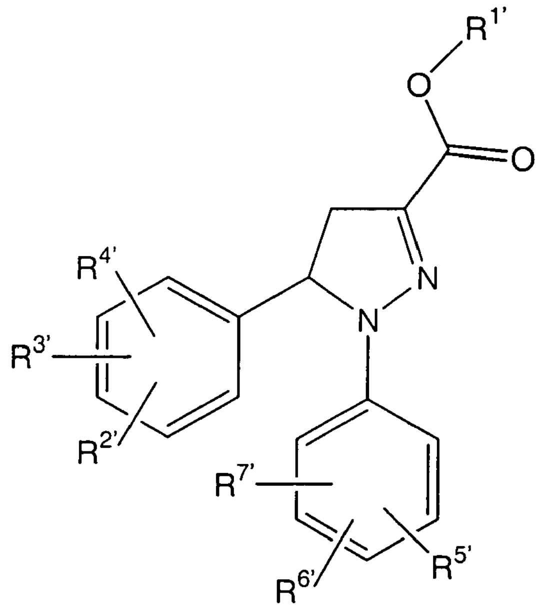

I’

wherein

$R^{1'}$ represents hydrogen or a linear or branched $C_{1-4}$-alkyl group,

$R^{2'}$, $R^{3'}$ and $R^{4'}$ independently of each other represent hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{8'}$, SH, $SR^{8'}$, $SOR^{8'}$, $SO_2R^{8'}$, $NH_2$, $NHR^{8'}$, $NR^{8'}R^{9'}$, -(C=O)-$NH_2$, -(C=O)-$NHR^{8'}$ or -(C=O)-$NR^{8'}R^{9'}$ whereby $R^{8'}$ and $R^{9'}$ for each substituent independently represent linear or branched $C_{1-6}$ alkyl,

$R^{5'}$ and $R^{6'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for

each substituent independently represent linear or branched $C_{1-6}$ alkyl;
$R^{7'}$ represents hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, - (C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof. and
(B) at least one lipid lowering compound used to treat dylipidaemia.

**2.** Active substance combination according to claim 1, **characterized in that** $R^1$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R" whereby R' and R" for each substituent independently represent linear or branched $C_{1-6}$ alkyl, preferably $R^1$ represents a phenyl group, which is optionally substituted by one or more substituents selected from the group consisting of methyl, ethyl, F, Cl, Br and $CF_3$, more preferably $R^1$ represents a phenyl group, which is mono-substituted with a chlorine atom in the 4-position.

**3.** Active substance combination according to claim 1 or 2, **characterized in that** $R^2$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R", whereby R' and optionally R" for each substituent independently represent linear or branched $C_{1-6}$ alkyl, preferably $R^2$ represents a phenyl group, which is optionally substituted by one or more substituents independently selected from the group consisting of methyl, ethyl, F, Cl, Br and $CF_3$, more preferably $R^2$ represents a phenyl group, which is di-substituted with two chlorine atoms in its 2- and 4-position.

**4.** Active substance combination according to one or more of claims 1-3, **characterized in that** $R^3$ represents a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or $R^3$ represents an optionally at least mono-substituted, 5- or 6-membered aryl or heteroaryl group, which may be condensed with an optionally at least mono-substituted mono- or polycyclic ring system, or $R^3$ represents an -$NR^4R^5$-moiety, preferably $R^3$ represents a saturated, optionally at least mono-substituted, optionally one or more nitrogen-atoms as ring member containing $C_{3-8}$ cycloaliphatic group, which may be condensed with an optionally at least mono-substituted mono- or polycyclic ring system, or $R^3$ represents an - $NR^4R^5$-moiety, more preferably $R^3$ represents a pyrrolidinyl group, a piperidinyl group or a piperazinyl group, whereby each of these groups may be substituted with one or more $C_{1-6}$-alkyl groups, or $R^3$ represents an - $NR^4R^5$-moiety.

**5.** Active substance combination according to one or more of claims 1-4, **characterized in that** $R^4$ and $R^5$, identical or different, represent a hydrogen atom; an unbranched or branched, saturated or unsaturated, optionally at least mono-substituted $C_{1-6}$-aliphatic radical; a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system and/or bonded via a methylene (-$CH_2$-) or ethylene (-$CH_2$-$CH_2$)-group; an-$SO_2$-$R^6$-moiety; or an -$NR^7R^8$-moiety, preferably one of these residues $R^4$ and $R^5$ represents a hydrogen atom and the other one of these residues $R^4$ and $R^5$ represents a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system; an -$SO_2$-$R^6$-moiety; or an - $NR^7R^8$-moiety, or $R^4$ and $R^5$, identical or different, each represent a $C_{1-6}$ alkyl group, more preferably one of these residues $R^4$ and $R^5$ represents a hydrogen atom and the other one of these residues $R^4$ and $R^5$ represents an optionally at least mono-substituted pyrrolidinyl group; an optionally at least mono-substituted piperidinyl group; an optionally at least mono-substituted piperazinyl group; an optionally at least mono-substituted triazolyl group; an - $SO_2$-$R^6$-moiety; or an -$NR^7R^8$-moiety, or $R^4$ and $R^5$, identical or different, represent a methyl group, an ethyl group, an n-propyl group, an isopropyl group, an n-butyl

group, a sec-butyl group or a tert.-butyl group.

6. Active substance combination according to one or more of claims 1-5, **characterized in that** $R^6$ represents a linear or branched, saturated or unsaturated, optionally at least mono-substituted $C_{1-6}$ aliphatic group; a saturated or unsaturated, optionally at least mono-substituted, optionally at least one heteroatom as ring member containing $C_{3-8}$ cycloaliphatic group, which may be condensed with a mono- or polycyclic ring-system; or an optionally at least mono-substituted, 5- or 6-membered aryl or heteroaryl group, which may be condensed with a mono- or polycyclic ring system and/or bonded via a methylene ($-CH_2-$) or ethylene ($-CH_2-CH_2$)-group, preferably $R^6$ represents a $C_{1-6}$-alkyl group; a saturated, optionally at least mono-substituted cycloaliphatic group, which may be condensed with a mono- or polycyclic ring-system; or a phenyl group, which is optionally substituted with one or more $C_{1-6}$ alkyl groups.

7. Active substance combination according to one or more of claims 1-6, **characterized in that** $R^7$ and $R^8$, identical or different, represent a hydrogen atom; an unbranched or branched, saturated or unsaturated, optionally at least mono-substituted $C_{1-6}$ aliphatic radical; a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system; or an optionally at least mono-substituted, 5- or 6 membered aryl or heteroaryl group, which may be condensed with an optionally at least mono-substituted mono- or polycyclic ring system and/or bonded via a methylene ($-CH_2-$) or ethylene ($-CH_2-CH_2$)-group, preferably $R^7$ and $R^8$, identical or different, represent a hydrogen atom or a $C_{1-6}$ alkyl radical.

8. Active substance combination according to one or more of claims 1-7, **characterized in that**
$R^1$ represents a phenyl group, which is optionally substituted with 1, 2, 3, 4 or 5 substituents independently selected from the group consisting of methyl, ethyl, F, Cl, Br and $CF_3$,
$R^2$ represents a phenyl group, which is optionally substituted with 1, 2, 3, 4 or 5 substituents independently selected from the group consisting of methyl, ethyl, F, Cl, Br and $CF_3$,
$R^3$ represents a pyrrolidinyl group, a piperidinyl group or a piperazinyl group, whereby each of these groups may be substituted with one or more of $C_{1-6}$-alkyl groups, or $R^3$ represents an $-NR^4R^5$-moiety,
$R^4$ represents a hydrogen atom or a linear or branched $C_{1-6}$-alkyl group,
$R^5$ represents a linear or branched $C_{1-6}$ alkyl group; an $-SO_2-R^6$-moiety; a pyrrolidinyl group; a piperidinyl group; a piperazinyl group; a homo-piperazinyl group; a morpholinyl group; a triazolyl group; whereby each of the heterocyclic rings may be substituted with one or more, identical or different, $C_{1-6}$-alkyl groups, and
$R^6$ represents a phenyl group, which is optionally substituted with one or more $C_{1-6}$ alkyl groups, which may be identical or different.

9. Active substance combination according to one or more of claims 1-8, **characterized in that**
$R^1$ represents a phenyl ring, which is mono-substituted with a halogen atom, preferably a chlorine atom, in its 4-position,
$R^2$ represents a phenyl ring, which is di-substituted with two halogen atoms, preferably chlorine atoms, in its 2- and 4-position,
$R^3$ represents a pyrrolidinyl group, a piperidinyl group, a piperazinyl group, a homo-piperazinyl group, a morpholinyl group, or an $-NR^4R^5$-moiety,
$R^4$ represents a hydrogen atom or a linear or branched $C_{1-6}$-alkyl group,
$R^5$ represents a linear or branched $C_{1-6}$ alkyl group; an $-SO_2-R^6$-moiety; a pyrrolidinyl group; a piperidinyl group; a piperazinyl group; a homo-piperazinyl group; a morpholinyl group; or a triazolyl group whereby each of the heterocyclic rings may be substituted with one or more, identical or different, $C_{1-6}$-alkyl groups, and
$R^6$ represents a phenyl group, which is optionally substituted with one or more $C_{1-6}$ alkyl groups, which may be identical or different.

10. Active substance combination according to one or more of claims 1-9, **characterized in that** the pyrazoline compound of general formula I is selected from the group consisting of
N-piperidinyl-5-(4-chloro-phenyl)-1-(2,4-dichlorophenyl)-4,5-dihydro-1 H-pyrazol-3-carboxamide,
5-(4-Chloro-phenyl)-1-(2,4-dichloro-phenyl)-4,5-dihydro-1 H-pyrazole-3-carboxylic acid-[1,2,4]-triazole-4-yl-amide,
5-(4-Chloro-phenyl)-1-(2,4-dichloro-phenyl)-4,5-dihydro-1 H-pyrazole-3-carboxylic acid-(4-methyl-piperazin-1-yl)-amide,
5-(4-Chloro-phenyl)-1-(2,4-dichloro-phenyl)-4,5-dihydro-1 H-pyrazole-3-carboxylic acid diethylamide,
[5-(4-Chloro-phenyl)-1-(2,4-dichloro-phenyl)-4,5-dihydro-1 H-pyrazole-3-yl]-piperidine-1-yl-methanone and
N-[5-(4-Chloro-phenyl)-1-(2,4-dichlorophenyl)-4,5-dihydro-1H-pyrazole-3-carbonyl]-4-methylphenylsulfonamide,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

**11.** Active substance combination according to one or more of claims 1-10, **characterized in that** the pyrazoline compound of general formula I is one of the following compounds

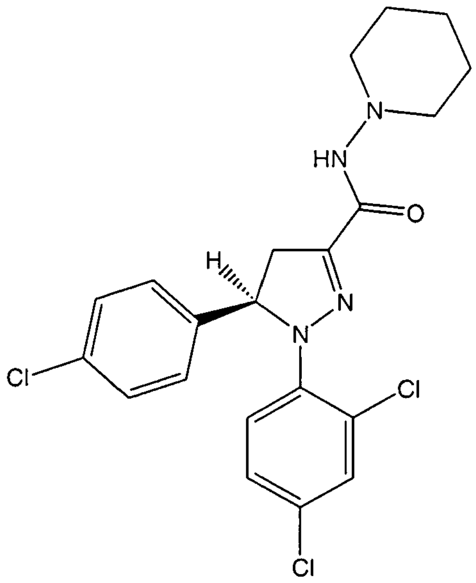

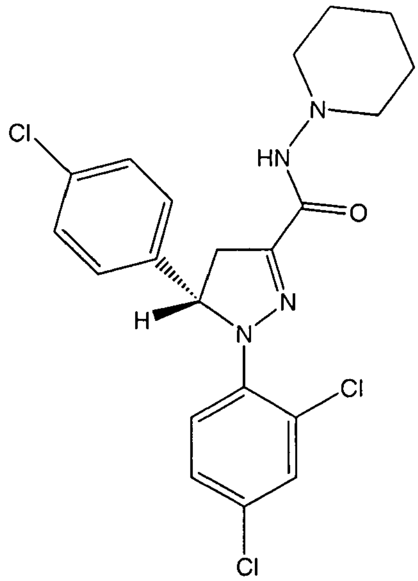

in each case optionally in the form of a corresponding N-oxide, a corresponding salt or a corresponding solvate thereof.

**12.** Active substance combination according to one or more of claims 1-11, **characterized in that** at least one of $R^{2'}$, $R^{3'}$ or $R^{4'}$ represents hydrogen, while at least one of $R^{2'}$, $R^{3'}$ or $R^{4'}$ is different from hydrogen.

**13.** Active substance combination according to one or more of claims 1-12, **characterized in that** $R^{7'}$ represents hydrogen.

**14.** Active substance combination according to one or more of claims 1-13, **characterized in that** $R^{2'}$, $R^{3'}$ and $R^{4'}$ independently of each other represent hydrogen, a linear or branched $C_{1-6}$-alkyl group, a halogen atom, or $CF_3$, preferably $R^{2'}$, $R^{3'}$ and $R^{4'}$ independently of each other represent hydrogen, methyl, ethyl, F, Cl, Br and $CF_3$.

**15.** Active substance combination according to one or more of claims 1-14, **characterized in that** $R^{5'}$ and $R^{6'}$ independently of each other represent a linear or branched $C_{1-6}$-alkyl group, a halogen atom, or $CF_3$, preferably $R^{5'}$ and $R^{6'}$ independently of each other represent methyl, ethyl, F, Cl, Br and $CF_3$.

**16.** Active substance combination according to one or more of claims 1-15, **characterized in that** $R^{2'}$ represents a chlorine atom in the 4-position of the phenyl ring, while $R^{3'}$ and $R^{4'}$ represent hydrogen.

**17.** Active substance combination according to one or more of claims 1-16, **characterized in that** $R^{5'}$ and $R^{6'}$ each represent a chlorine atoms in the 2- and 4-position of the phenyl ring, while $R^{7'}$ represents hydrogen.

**18.** Active substance combination according to one or more of claims 1-17, **characterized in that** $R^{1'}$ represents hydrogen, methyl or ethyl, preferably hydrogen.

**19.** Active substance combination according to one or more of claims 1-18, **characterized in that** the compounds of general formula I' are represented by the following structure II:

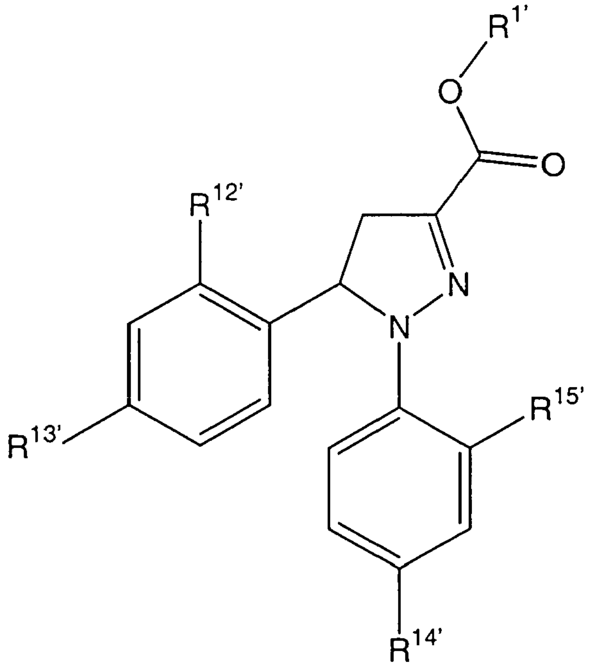

II

wherein
$R^{1'}$ represents hydrogen or a linear or branched $C_{1-4}$-alkyl group,
$R^{12'}$ or $R^{13'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$,
$R^{14'}$ or $R^{15'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, methyl, ethyl, F, Cl, Br and $CF_3$,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

**20.** Active substance combination according to claim 19, **characterized in that** $R^{12'}$ and $R^{13'}$ independently of each other represent hydrogen, a linear or branched $C_{1-6}$-alkyl group, a halogen atom, or $CF_3$, preferably $R^{12'}$ and $R^{13'}$ independently of each other represent hydrogen, methyl, ethyl, F, Cl, Br and $CF_3$.

**21.** Active substance combination according to claim 19 or 20, **characterized in that** $R^{14'}$ and $R^{15'}$ independently of each other represent a linear or branched $C_{1-6}$-alkyl group, a halogen atom, or $CF_3$, preferably $R^{14'}$ and $R^{15'}$ independently of each other represent methyl, ethyl, F, Cl, Br and $CF_3$.

**22.** Active substance combination according to one or more of claims 19-21, **characterized in that** $R^{13'}$ represents Cl and $R^{12'}$ represents hydrogen.

**23.** Active substance combination according to one or more of claims 19-22, **characterized in that** $R^{14'}$ and $R^{15'}$ each represent Cl.

**24.** Active substance combination according to one or more of claims 19-23, **characterized in that** $R^{1'}$ represents hydrogen, methyl or ethyl, preferably hydrogen.

**25.** Active substance combination according to one or more of claims 1-24, **characterized in that** the compound of general formula I' is
5-(4-chloro-phenyl)-1-(2,4-dichlorophenyl)-4,5-dihydro-1H-pyrazol-3-carboxylic acid,
optionally in form of one of the stereoisomers, preferably enantiomers or diastereomers, a racemate or in form of a

mixture of at least two of the stereoisomers, preferably enantiomers and/or diastereomers, in any mixing ratio, or a corresponding salt thereof, or a corresponding solvate thereof.

**26.** Active substance combination according to one or more of claims 1-25, **characterized in that** the pyrazoline compound of general formula I' is one of the following compounds

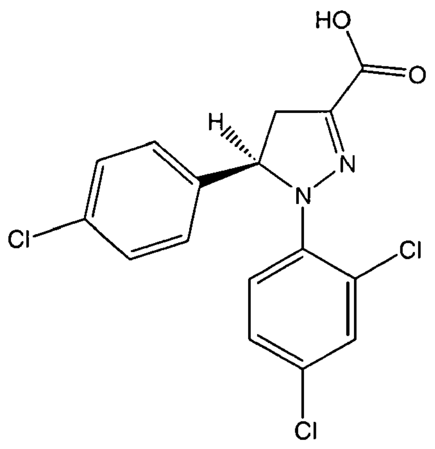

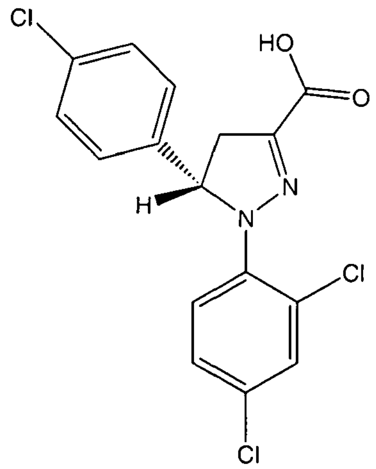

in each case optionally in the form of a corresponding N-oxide, a corresponding salt or a corresponding solvate thereof.

**27.** Use of an active substance combination according to one or more of claims 1-26, for the manufacture of a medicament for the treatment of dyslipidaemia and/or obesity and/or apetancy disorders and/or pre-diabetes or type 2 diabetes and/or drug-induced weight gain or drug-induced obesity.

**28.** Use qccording to claim 27, wherein the statin compound of component (B) is selected from the group consisting of atorvastatin, rosuvastatin, simvastatin, pravastatin, cerivastatin, mevastatin, velostatin, fluvastatin, compactin, dihydrocompactin, lovastatin, dalvastatin and fluindostatin, optionally in the form of a corresponding salt or a corresponding solvate thereof.

**29.** Use according to claim 27 wherein the statin compound of component (B) is selected from the group consisting of atorvastatin, atorvastatin-calcium, rosuvastatin, rosuvastatin-sodium, simvastatin, pravastatin, pravastatin-sodium, cerivastatin, cerivastatin-sodium, mevastatin, velostatin, fluvastatin, fluvastatin-sodium, compactin, dihydrocompactin, lovastatin, dalvastatin and fluindostatin.

**30.** Use according to claim 27 wherein the active substance combination comprises

(A) at least one substituted pyrazoline compound of general formula I

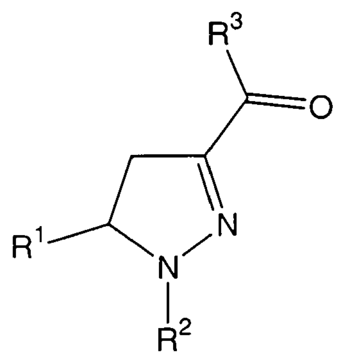

I

wherein
$R^1$ represents a phenyl group, which is optionally substituted with 1, 2, 3, 4 or 5 substituents independently selected from the group consisting of methyl, ethyl, F, Cl, Br and $CF_3$,
$R^2$ represents a phenyl group, which is optionally substituted with 1, 2, 3, 4 or 5 substituents independently selected from the group consisting of methyl, ethyl, F, Cl, Br and $CF_3$,
$R^3$ represents a pyrrolidinyl group, a piperidinyl group or a piperazinyl group, whereby each of these groups may be substituted with one or more of $C_{1-6}$-alkyl groups, or $R^3$ represents an $-NR^4R^5$-moiety,
$R^4$ represents a hydrogen atom or a linear or branched $C_{1-6}$-alkyl group,
$R^5$ represents a linear or branched $C_{1-6}$ alkyl group; an $-SO_2-R^6$-moiety; a pyrrolidinyl group; a piperidinyl group; a piperazinyl group; a homo-piperazinyl group; a morpholinyl group; a triazolyl group; whereby each of the heterocyclic rings may be substituted with one or more, identical or different, $C_{1-6}$-alkyl groups, and
$R^6$ represents a phenyl group, which is optionally substituted with one or more $C_{1-6}$ alkyl groups, which may be identical or different.
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and/or
at least one substituted pyrazoline compound of general formula I' represented by the following structure II:

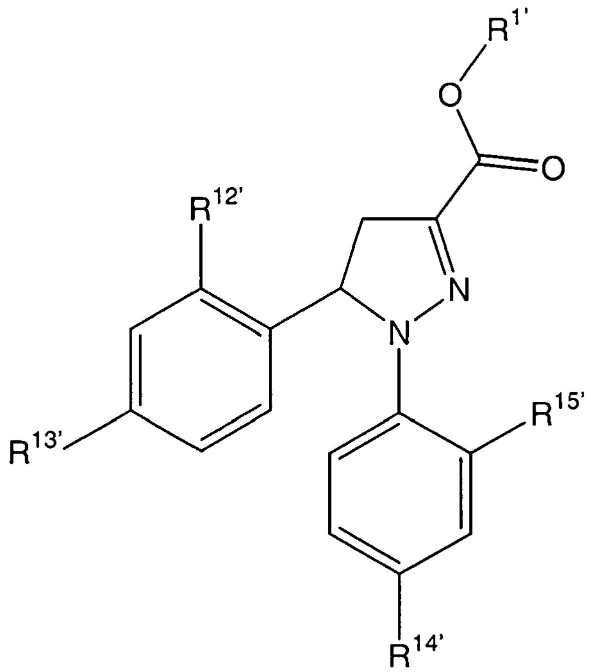

II

wherein
$R^{1'}$ represents hydrogen or a linear or branched $C_{1-4}$-alkyl group, $R^{12'}$ or $R^{13'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$,
$R^{14'}$ or $R^{15'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, methyl, ethyl, F, Cl, Br and $CF_3$,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

and
(B) at least one statin compound selected from the group consisting of atorvastatin, atorvastatin-calcium, rosuvastatin, rosuvastatin-sodium, simvastatin, pravastatin, pravastatin-sodium, cerivastatin, cerivastatin-sodium, mevastatin, velostatin, fluvastatin, fluvastatin-sodium, compactin, dihydrocompactin, lovastatin, dalvastatin and fluindostatin.

**31.** Use according to claim 27 wherein the active substance combination according comprises

(A) one or more substituted pyrazoline compounds of general formula I selected from the group consisting of
N-piperidinyl-5-(4-chloro-phenyl)-1-(2,4-dichlorophenyl)-4,5-dihydro-1H-pyrazol-3-carboxamide,
5-(4-Chloro-phenyl)-1-(2,4-dichloro-phenyl)-4,5-dihydro-1H-pyrazole-3-carboxylic acid-[1 ,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone and
N-[5-(4-Chloro-phenyl)-1-(2,4-dichlorophenyl)-4,5-dihydro-1H-pyrazole-3-carbonyl]-4-methylphenylsulfonamide,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,
and/or
5-(4-chloro-phenyl)-1-(2,4-dichlorophenyl)-4,5-dihydro-1H-pyrazol-3-carboxylic acid
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof,
and
(B) at least one statin compound selected from the group consisting of atorvastatin, atorvastatin-calcium, rosuvastatin, rosuvastatin-sodium, simvastatin, pravastatin, pravastatin-sodium, cerivastatin, cerivastatin-sodium, mevastatin, velostatin, fluvastatin, fluvastatin-sodium, compactin, dihydrocompactin, lovastatin, dalvastatin and fluindostatin.

**32.** Active substance combination according to one or more of claims 1-26, **characterized in that** the molar ratio of component (A) to component (B) is in the range of 1:10 to 10:1, preferably 1:5 to 5:1.

**33.** Medicament comprising an active substance combination according to one or more of claims 1 to 26, wherein component (B) is not a statin, and optionally at least one further active substance and/or optionally at least one auxiliary substance.

**34.** Medicament according to claim 33 for the modulation of cannabinoid-receptors, preferably cannabinoid 1 ($CB_1$) receptors; and/or for HMG-CoA reductase inhibition.

**35.** Medicament according to claim 33 or 34 for the prophylaxis and/or treatment of coronary heart disease, myocardial ischemia, angina pectoris, atherosclerosis, hypertension, hyperlipidemia, Lipoprotein Disorders, Hypercholesterolemia, or for lowering of cardiac risk.

**36.** Use of at least one active substance combination according to one or more of claims 1 to 26, wherein component (B) is not a statin, and optionally at least one further active substance and/or optionally at least one auxiliary substance for the manufacture of a medicament for the modulation of cannabinoid-receptors, preferably cannabinoid 1 ($CB_1$) receptors; and/or HMG-CoA reductase inhibition.

**37.** Use of at least one active substance combination according to one or more of claims 1 to 26, wherein component (B) is not a statin, and optionally at least one further active substance and/or optionally at least one auxiliary substance for the manufacture of a medicament for the prophylaxis and/or treatment of coronary heart disease, myocardial ischemia, angina pectoris, atherosclerosis, hypertension, hyperlipidemia, Hypercholesterolemia, or for lowering of cardiac risk.

**38.** Pharmaceutical formulation comprising an active substance combination according to one or more of claims 1 to

26, wherein component (B) is not a statin, and optionally at least one further active substance and/or optionally at least one auxiliary substance.

**39.** Pharmaceutical formulation according to claim 38, **characterized in that** it is suitable for oral or parenteral administration, preferably for oral, intravenous, intraperitoneal, intramuscular, subcutaneous, intrathekal, rectal, transdermal, transmucosal or nasal administration.

**40.** Pharmaceutical formulation for oral administration according to claim 39, **characterized in that** it is in the form of a tablet, a drageé, a powder, a capsule, drops, a gel, juice, sirup, chewing gum, solution or suspension.

**41.** Pharmaceutical formulation for oral administration according to claim 39, **characterized in that** it is in form of multiparticulates, preferably microparticles, microtablets, pellets or granules, optionally compressed into a tablet, filled into a capsule or suspended in a suitable liquid.

**42.** Pharmaceutical formulation for oral administration according to claims 39-41, **characterized in that** it comprises at least one enteric coating.

**43.** Pharmaceutical formulation according to claim 38-42 **characterized in that** it comprises component (A) and/or component (B) at least partially in a sustained-release form.

**44.** Pharmaceutical formulation according to claim 43, **characterized in that** the sustained release is achieved by at least one coating or matrix comprising at least one sustained-release material.

**45.** Pharmaceutical formulation according to claim 44, **characterized in that** the sustained-release material is based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.

**46.** Pharmaceutical formulation according to claim 45, **characterized in that** the water-insoluble polymer is based on an acrylic resin, which is preferably selected from the group of poly(meth)acrylates, poly($C_{1-4}$)dialkylamino($C_{1-4}$) alkyl (meth)acrylates and/or copolymers thereof or a mixture of at least two of the afore-mentioned polymers.

**47.** Pharmaceutical formulation according to claim 45, **characterized in that** the water-insoluble polymers are cellulose derivatives, preferably alkyl cellulose, particularly preferably ethyl cellulose, or cellulose esters.

**48.** Pharmaceutical formulation according to claim 45, **characterized in that** the wax is carnauba wax, beeswax, glycerol monostearate, glycerol monobehenate, glycerol ditripalmitostearate, microcrystalline wax or a mixture of at least two of these components.

**49.** Pharmaceutical formulation according to any one of claims 45-48, **characterized in that** the polymers have been used in combination with one or more plasticizers.

**50.** Pharmaceutical formulation according to any one of claims 38-49, **characterized in that** it comprises component (A) and/or (B) in immediate-release form as well as in sustained release form.

**51.** A method of modulating cannabinoid-receptors, preferably cannabinoid 1 ($CB_1$) receptors; and/or for inhibiting HMG-CoA reductase, comprising administering to a subject, preferably a human, a therapeutically effective amount of an active substance combination according to one or more of claims 1-31.

**52.** A method of treating one or more diseases selected from the group consisting of coronary heart disease, myocardial ischemia, angina pectoris, atherosclerosis, hypertension, hyperlipidemia, Lipoprotein Disorders and Hypercholesterolemia, comprising administering to a subject, preferably a human, a therapeutically effective amount of an active substance combination according to one or more of claims 1-26, wherein component (B) is not a statin.

**53.** A method for lowering of cardiac risk, comprising administering to a subject, preferably a human, a therapeutically effective amount of an active substance combination according to one or more of claims 1-26 wherein component (B) is not a statin.

European Patent Office

# PARTIAL EUROPEAN SEARCH REPORT

which under Rule 45 of the European Patent Convention shall be considered, for the purposes of subsequent proceedings, as the European search report

Application Number
EP 07 38 4009

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/074920 A (SOLVAY PHARM BV [NL]; LANGE JOSEPHUS H M [NL]; KRUSE CORNELIS G [NL];) 18 August 2005 (2005-08-18)<br>* page 11, lines 15-38; claims 9-12; compounds 1-4 *<br>----- | 1-25,27, 32-45, 51-53 | INV.<br>A61K45/06<br>A61P3/06 |
| X | WO 2005/077909 A (ESTEVE LABOR DR [ES]; CUBERES ALTISEN ROSA [ES]; GUITIERREZ SILVA BONI) 25 August 2005 (2005-08-25)<br>* page 54; claims *<br>----- | 1-53 | |
| Y | FR 2 861 303 A (SANOFI SYNTHELABO [FR]) 29 April 2005 (2005-04-29)<br>* page 3, lines 8-18 *<br>* page 4, lines 14-25 *<br>* page 6, lines 7-28; claims *<br>-----<br>-/-- | 1-53 | |

TECHNICAL FIELDS SEARCHED (IPC)

A61K
A61P

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

3

| Place of search | Date of completion of the search | Examiner |
|---|---|---|
| Munich | 8 June 2007 | ESCOLAR BLASCO, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

# PARTIAL EUROPEAN SEARCH REPORT

Application Number
EP 07 38 4009

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| Y | LANGE J H M ET AL: "BIOISOSTERIC REPLACEMENTS OF THE PYRAZOLE MOIETY OF RIMONABANT: SYNTHESIS, BIOLOGICAL PROPERTIES, AND MOLECULAR MODELING INVESTIGATIONS OF THIAZOLES, TRIAZOLES, AND IMIDAZOLES AS POTENT AND SELECTIVE CB1 CANNABINOID RECEPTOR ANTAGONISTS" JOURNAL OF MEDICINAL CHEMISTRY, AMERICAN CHEMICAL SOCIETY. WASHINGTON, US, vol. 48, 2005, pages 1823-1838, XP002339942 ISSN: 0022-2623 * page 1828, right-hand column; figure 1 * ----- | 1-53 | |
| | | | TECHNICAL FIELDS SEARCHED (IPC) |
| A | FOR THE RIO-EUROPE STUDY GROUP VAN GAAL L F ET AL: "Effects of the cannabinoid-1 receptor blocker rimonabant on weight reduction and cardiovascular risk factors in overweight patients: 1-year experience from the RIO-Europe study" LANCET THE, LANCET LIMITED. LONDON, GB, vol. 365, no. 9468, 16 April 2005 (2005-04-16), pages 1389-1397, XP004849986 ISSN: 0140-6736 * abstract * * page 1395, left-hand column, paragraph 2 * ----- | 1-53 | |
| E | WO 2007/009698 A (ESTEVE LABOR DR [ES]; BUSCHMANN HELMUT H [ES]) 25 January 2007 (2007-01-25) * claims * ----- | 1-32,51 | |

3

EPO FORM 1503 03.82 (P04C10)

European Patent Office

INCOMPLETE SEARCH SHEET C

Application Number
EP 07 38 4009

Claim(s) not searched:
-

Reason for the limitation of the search (non-patentable invention(s)):

Claims 51-53: Article 52 (4) EPC - Method for treatment of the human or animal body by surgery

## ANNEX TO THE EUROPEAN SEARCH REPORT ON EUROPEAN PATENT APPLICATION NO.

EP 07 38 4009

This annex lists the patent family members relating to the patent documents cited in the above-mentioned European search report.
The members are as contained in the European Patent Office EDP file on
The European Patent Office is in no way liable for these particulars which are merely given for the purpose of information.

08-06-2007

| Patent document cited in search report | | Publication date | Patent family member(s) | | | Publication date |
|---|---|---|---|---|---|---|
| WO 2005074920 | A | 18-08-2005 | AU | 2005210163 | A1 | 18-08-2005 |
| | | | CA | 2552940 | A1 | 18-08-2005 |
| | | | KR | 20060131861 | A | 20-12-2006 |
| | | | MX | PA06008593 | A | 28-08-2006 |
| WO 2005077909 | A | 25-08-2005 | AU | 2005212817 | A1 | 25-08-2005 |
| | | | CA | 2556568 | A1 | 25-08-2005 |
| | | | KR | 20060135815 | A | 29-12-2006 |
| FR 2861303 | A | 29-04-2005 | AR | 047764 | A1 | 22-02-2006 |
| | | | AU | 2004289078 | A1 | 26-05-2005 |
| | | | BR | PI0415538 | A | 26-12-2006 |
| | | | CA | 2543582 | A1 | 26-05-2005 |
| | | | EP | 1680117 | A2 | 19-07-2006 |
| | | | WO | 2005046689 | A2 | 26-05-2005 |
| | | | JP | 2007509113 | T | 12-04-2007 |
| | | | KR | 20060100443 | A | 20-09-2006 |
| | | | US | 2007072907 | A1 | 29-03-2007 |
| WO 2007009698 | A | 25-01-2007 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4444784 A
- US 4346227 A
- US 5502199 A
- EP 617019 A
- EP 0325130 A
- US 3983140 A
- US 4448784 A
- US 4450171 A
- US 4739073 A
- EP 0244364 A
- US 4804770 A
- US 4231938 A
- EP 0306210 A
- EP 738510 A
- EP 363934 A
- US 4681893 A
- US 5273995 A
- US 5260440 A


### Non-patent literature cited in the description


- **ZEPHIER et al.** *International Diabetes Federation,* 1997
- *International Diabetes Federation,* 2005
- *Synthetic communications,* 1996, vol. 26 (11), 2229-33
- *Synlett,* 2001, (1), 147-149
- **PASCUAL, A.** *J. Prakt Chem.,* 1999, vol. 341 (7), 695-700
- **LIN, S. et al.** *Heterocycles,* 2001, vol. 55 (2), 265-277
- **RAO, P. et al.** *J. Org. Chem.,* 2000, vol. 65 (22), 7323-7344
- **PEARSON D.E ; BUEHLER, C.A.** *Synthesis,* 1972, 533-542
- **KROGSGAARD-LARSEN et al.** Textbook of Drugdesign and Discovery. Taylor & Francis, April 2002
- **WILSON et al.** *Am. J. Cardiol.,* 1987, vol. 59 (14), 91G-94G
- Pharmaceutics: the Science of Dosage Forms. Churchill Livingstone, 2002
- Encyclopedia of Pharmaceutical Technology. Marcel Dekker, Inc, 2002
- Modern Pharmaceutics. Marcel Dekker, Inc, 2002
- The Theory and Practice of Industrial Pharmacy. Lea & Febiger, 1986
- Modified-Release Drug Delivery Technology. Marcel Dekker, Inc, 2002
- Handbook of Pharmaceutical Controlled Release Technology. Marcel Dekker, Inc, 2000
- Controlled Drug Delivery. CRC Press Inc, 1983, vol. I
- Oral Drug delivery. **TAKADA, K. ; YOSHIKAWA, H.** Encyclopedia of Controlled Drug Delivery. John Wiley & Sons, Inc, 1999, vol. 2, 728-742
- Oral drug delivery, small intestine and colon. **FIX, J.** Encylopedia of Controlled Drug Delivery. John Wiley & Sons, Inc, 1999, vol. 2, 698-728
- Pharmaceutical tablet coating. **JOHNSON, J.L.** Coatings Technology Handbook. Marcel Dekker, Inc, 2001, 863-866
- **CARSTENSEN, T.** Coating Tablets in Advanced Pharmaceutical Solids. Marcel Dekker, Inc, 2001, 455-468
- **LEOPOLD, C.S.** Coated dosage forms for colon-specific drug delivery. *Pharmaceutical Science & Technology Today,* 1999, vol. 2 (5), 197-204
- **RHODES, C.T. ; PORTER, S.C.** Coatings, in Encyclopedia of Controlled Drug Delivery. John Wiley & Sons, Inc, 1999, vol. 1, 299-311